# EUROPEAN PATENT SPECIFICATION

(11) **EP 3 085 357 B1**
(45) Date of publication and mention of the grant of the patent: **13.02.2019**
(21) Application number: 14870738.3
(22) Date of filing: 15.12.2014
(51) Int. Cl.: A61K 9/00, A61K 39/00, A61K 39/12, A61K 39/145, A61K 47/10, A61K 47/24, A61K 47/26, A61K 47/36

(54) **MICRONEEDLE**
MIKRONADEL
MICROAIGUILLE

(30) Priority: 16.12.2013 JP 2013259672
(43) Date of publication of application: 26.10.2016
(73) Proprietor: Takeda Pharmaceutical Company Limited, Osaka-shi, Osaka 541-0045 (JP)
(72) Inventor: TANOUE, Yutaka, Fujisawa-shi, Kanagawa 251-0012 (JP); ISHII, Yumiko, Osaka-shi Osaka 532-0024 (JP); OMACHI, Yoshihiro, Osaka-shi Osaka 532-0024 (JP); MANOSHIRO, Tomoyuki, Osaka-shi Osaka 532-0024 (JP)
(74) Representative: Huenges, Martin
(86) International application number: PCT/JP2014/083184
(87) International publication number: WO 2015/093452

(56) References cited:
- EP-A1- 2 679 242
- WO-A1-2005/002453
- WO-A1-2009/066763
- WO-A1-2010/040271
- WO-A2-2008/130587
- JP-A- 2008 507 590
- JP-A- 2012 041 329
- JP-A- 2013 075 165
- JP-A- 2013 189 432
- JP-A- 2014 004 077
- US-A1- 2014 276 378

## Description

### Technical Field

The present invention relates to a microneedle.

### Background of Invention

Administration of a vaccine antigen in the form of a liquid or a freeze-dried preparation has been conventionally studied (Patent Literatures 5, 7 and 8), and is mainly performed by injection. The injection is, however, a painful administration method in which, for example, pain is felt in pricking the skin with an injection needle. Here, a freeze-dried preparation is a preparation generally known as a porous dried product or a powder, and does not have a puncturing property against a body surface such as the skin.

On the other hand, as an administration method in which pain is remarkably reduced, a method using a microneedle is drawing attention. A microneedle refers to a preparation having a refined needle. If a microneedle containing a drug is applied to a body surface such as the skin, the refined needle sticks in the body surface so as to administer the drug into the body surface (Patent Literature 2). In addition, there is a report that an antigen is produced through intradermal administration although a preparation is not disclosed (Non Patent Literature 1). Since a needle is refined in a microneedle, the needle sticking in a body surface is less likely to cause pain. Accordingly, a method for painlessly administering a vaccine antigen by using a microneedle containing the vaccine antigen has been provided.

As the form of a microneedle, various forms such as one having a drug coated on the surface of a refined needle, and one having a drug contained in a refined needle have been developed. For the form of a microneedle having a drug contained in a refined needle, a production method specialized for forming the needle has been developed.

Some examples of a drug-coated microneedle have been already known (Non Patent Literature 2, Non Patent Literature 3 and Patent Literature 6). On the other hand, as a microneedle improved in the operability, the safety and the certainty in drug administration, a soluble microneedle is drawing attention. A soluble microneedle refers to a microneedle in which a needle is formed by mixing a drug and a base material and the drug is dissolved together with the base material inside a body after stuck. Some examples of such a soluble microneedle have been also developed (Patent Literature 1, Patent Literature 3 and Patent Literature 4).

### Citation List

### Patent Literature

Patent Literature 1: International Publication WO2009/066763
Patent Literature 2: International Publication WO1996/003978
Patent Literature 3: International Publication WO2008/130587
Patent Literature 4: JP 2012-41329 A
Patent Literature 5: International Publication WO2008/042789
Patent Literature 6: International Publication WO2006/055799
Patent Literature 7: International Publication WO2013/009849
Patent Literature 8: JP 2010-539192 A

### Non Patent Literature

Non Patent Literature 1: Vaccine 29, (2009): 8126-8133
Non Patent Literature 2: Yeu-Chun Kim et al., J Control Release (2010), 142(2): 187-195
Non Patent Literature 3: Yeu-Chun Kim et al., Pharma Res (2011) 28: 135-144

### Summary of Invention

### Technical Problem

In a microneedle containing a vaccine antigen as a drug, however, the vaccine antigen is not always sufficiently stable.

In other words, there is a demand for development of a microneedle containing a stabilized vaccine antigen for making the microneedle more suitable to vaccine antigen administration. Solution to Problem

In order to solve such a problem, the present inventors made earnest studies on components contained in a microneedle, surprisingly found that the stability of a vaccine antigen is improved by mixing a specific component, and accomplished the present invention as a result of making further studies.

Specifically, the present invention relates to at least the following inventions:
[1] A preparation having a soluble microspike, the microspike containing a vaccine antigen, an ionic polymer base material, and at least one agent selected from the group consisting of a non-reducing sugar, a sugar alcohol, a cyclodextrin and a surfactant, wherein the ionic polymer base material contains a polysaccharide, and the polysaccharide is at least one selected from the group consisting of chondroitin sulfuric acid, hyaluronic acid, chitosan and chitin, and salts thereof, and wherein the surfactant is at least one selected from the group consisting of a nonionic surfactant and a lecithin.
[4] The preparation according to [1] above, in which an amount of the at least one selected from the group consisting of a non-reducing sugar, a sugar alcohol, a cyclodextrin and a surfactant is 0.01 to 94.99% by weight of the entire soluble microspike.
[5] The preparation according to [1] above, in which an amount of the vaccine antigen is 0.01 to 10% by weight of the entire soluble microspike.
[6] The preparation according to [1] above, in which an amount of the ionic polymer base material is 1 to 99.98% by weight of the entire soluble microspike.
[7] The preparation according to [1] above, in which degradation or aggregation occurring during drying or storage is suppressed to improve biological stability.
[8] The preparation according to [1] above, in which the soluble microspike has strength sufficient to be inserted, for use, into a body surface, and biologically stabilizes the vaccine antigen in a solid state.
[9] The preparation according to [1] above, in which the soluble microspike has in vivo solubility.
[10] The preparation according to [2] above, in which the vaccine antigen is a toxoid.
[11] The preparation according to [3] above, in which the vaccine antigen is a vaccine antigen having a particulate structure.
[12] The preparation according to [10] above, in which the toxoid is at least one selected from the group consisting of a tetanus toxoid and a diphtheria toxoid.
[13] The preparation according to [11] above, in which the vaccine antigen having a particulate structure is at least one selected from the group consisting of a norovirus vaccine, a Dengue fever vaccine, an HPV vaccine, an influenza vaccine and a rotavirus vaccine.
[14] The preparation according to [1] above, in which the ionic polymer base material is at least one selected from the group consisting of polysaccharides, copolymers thereof and salts thereof.
[16] The preparation according to [1] above, in which the non-reducing sugar and the sugar alcohol are at least one selected from the group consisting of trehalose, sucrose, mannitol and sorbitol.
[18] The preparation according to [1] above, in which the cyclodextrin is at least one selected from the group consisting of HP-β-cyclodextrin and G2-β-cyclodextrin.
[19] The preparation according to any one of [1] to [18] above, in which a projection including the soluble microspike is directly held on a support.
[20] The preparation according to any one of [1] to [18] above, in which a projection including the soluble microspike is held on a base to form a spike holding member, and the spike holding member is held on a support.
[21] A spike holding member used as a component of the preparation according to [20] above.
[22] A method for stabilizing a preparation having a soluble microspike, in which at least one selected from the group consisting of a non-reducing sugar, a sugar alcohol, a cyclodextrin and a surfactant, and an ionic polymer base material are contained in the soluble microspike containing a vaccine antigen.

### Advantageous Effects of Invention

If a preparation for a microneedle is prepared by mixing components defined by the present invention, an effect of improving the stability of an antigen contained in the microneedle can be exhibited. Besides, a microneedle of the present invention has an effect that an immune response to an antigen contained in the microneedle can be induced through intradermal administration.

### Brief Description of Drawings

Fig. 1 illustrates a micrograph, taken from a diagonally upper direction, of a microneedle patch produced in Example 1. Each microneedle is in the shape of a square pyramid having a base length of 300 µm and a height of 500 µm.
Fig. 2 illustrates a micrograph, taken from a diagonally upper direction, of a microneedle patch produced in Example 2. Each microneedle is in the shape of a square pyramid having a base length of 300 µm and a height of 500 µm.
Fig. 3 illustrates a micrograph, taken from a diagonally upper direction, of a microneedle patch produced in Example 3. Each microneedle is in the shape of a square pyramid having a base length of 300 µm and a height of 500 µm.
Fig. 4 illustrates a micrograph of a patch obtained in Comparative Example 1. Although a solid content was found on a patch surface, no needle was formed.
Fig. 5 illustrates results of detecting, through gel electrophoresis, a tetanus toxoid protein in samples immediately after preparation (initial sample) in Examples 4 to 6 and Comparative Example 2.
Fig. 6 illustrates results of detecting, through the gel electrophoresis, a tetanus toxoid protein in samples after stored at 40°C for 1 week after the preparation in Examples 4 to 6 and Comparative Example 2.
Fig. 7 illustrates micrographs of samples of a microneedle patch produced in Example 11 obtained immediately after preparation (initial sample) (upper row), after stored at 25°C for 1 month (middle row) and after stored at 40°C for 1 month (lower row). First and second micrographs in each row are photographs of the samples taken from above, and third and fourth in some of rows are photographs taken from a lateral direction.
Fig. 8 illustrates micrographs of samples of a patch produced in Comparative Example 6 obtained immediately after preparation (initial sample) (upper row), after stored at 25°C for 1 month (middle row) and after stored at 40°C for 1 month (lower row). First and second micrographs in each row are photographs of the samples taken from above, and third and fourth in some of rows are photographs taken from a lateral direction.
Fig. 9 illustrates micrographs, taken from a diagonally upper direction, of samples of a patch produced in Example 12 obtained after stored at 5°C for 3 months (upper row), after stored at 25°C and 60%RH for 3 months (middle row) and after stored at 40°C and 75%RH for 3 months (lower row).
Fig. 10 illustrates micrographs, taken from a diagonally upper direction, of samples of a patch produced in Example 13 obtained after stored at 5°C for 1 month (upper row), after stored at 25°C and 60%RH for 1 month (middle row) and after stored at 40°C and 75%RH for 1 month (lower row).
Fig. 11 illustrates micrographs, taken from a diagonally upper direction, of samples of a patch produced in Example 14 obtained after stored at 5°C for 1 month (upper row), after stored at 25°C and 60%RH for 1 month (middle row) and after stored at 40°C and 75%RH for 1 month (lower row).
Fig. 12 illustrates norovirus G1-specific immune response (upper row) and norovirus G2-specific immune response (lower row) induced in blood after administering the patches produced in Examples 12 to 14 to rabbits.
Fig. 13A is a conceptual diagram of one example of the shape of a preparation 1 of the present invention. A microspike 2 containing a vaccine antigen is directly bonded to a support 3 unified with a base and is in a shape projecting from the support 3 unified with the base. Specifically, the microspike 2 containing the vaccine antigen occupies the whole of a projection.
Fig. 13B is a conceptual diagram of another example of the shape of the preparation 1 of the present invention. A projection is in a shape projecting from a support 3 unified with a base. A microspike 2 forms a tip portion of the projection, and is bonded to the support 3 unified with the base via a projection base 4 not containing a vaccine antigen. Specifically, the projection includes a plurality of layers of a layer corresponding to the microspike 2 and containing the vaccine antigen and a layer corresponding to the projection base 4 and not containing the vaccine antigen.
Fig. 14 is a conceptual diagram of still another example of the shape of the preparation 1 of the present invention. A microspike 2 containing a vaccine antigen is directly bonded to a base 5 corresponding to a spike holding member and is in a shape projecting from the base 5. The base 5 is held on a support 6. Specifically, the preparation 1 is constituted by holding the microspike 2 on the support 6 with the base 5 sandwiched therebetween.

### Description of Embodiment

The present invention will now be described in detail.

A preparation having a soluble microspike, the microspike containing a vaccine antigen, an ionic polymer base material, and at least one agent selected from the group consisting of a non-reducing sugar, a sugar alcohol, a cyclodextrin and a surfactant, wherein the ionic polymer base material contains a polysaccharide, and the polysaccharide is at least one selected from the group consisting of chondroitin sulfuric acid, hyaluronic acid, chitosan and chitin, and salts thereof, and wherein the surfactant is at least one selected from the group consisting of a nonionic surfactant and a lecithin.

Herein, the preparation refers to a product in which a projection corresponding to a portion sticking in a body surface such as the skin is held on a support in the shape of a sheet, a tape, a plate, a block or the like. The projection may directly be held on the support unified with a base in some cases. Alternatively, the projection is held on the support with the base sandwiched therebetween, namely, the projection is held on the base and the base is held on the support, a member different from the base, in some cases. In the latter cases, the base holding the projection is sometimes designated as a spike holding member. The soluble microspike (herein, sometimes referred to also as the "microspike") refers to a portion containing the vaccine antigen and corresponding to the whole or a part of the projection. If the microspike corresponds to a tip portion of the projection and a base portion of the projection does not contain the vaccine antigen, the base portion of the projection not containing the vaccine antigen may specifically be designated as a projection base in some cases.

In the present invention, a sugar is any of monosaccharides, and oligosaccharides such as disaccharides, trisaccharides and tetrasaccharides. In the present invention, the non-reducing sugar is preferably an oligosaccharide, and examples include, but are not limited to, trehalose, sucrose, galactosucrose, trehalosamine, maltitol, cellobionic acid, lactobionic acid, lactitol and sucralose. Preferable examples of the non-reducing sugar include trehalose and sucrose.

In the present invention, examples of the sugar alcohol include, but are not limited to, mannitol, sorbitol, glycerol, erythritol, threitol, ribitol, arabinitol, xylitol, alitol, glucitol, iditol, galactitol and talitol. Preferable examples of the sugar alcohol include mannitol, sorbitol, glycerol, xylitol and erythritol, and further preferable examples include mannitol, sorbitol and glycerol.

Herein, the term "cyclodextrin" encompasses, in addition to a cyclodextrin, a derivative of the cyclodextrin, and salts of the cyclodextrin and the derivative thereof. Examples of the derivative of the cyclodextrin include hydroxypropyl-cyclodextrin, maltosyl-cyclodextrin, carboxymethyl-cyclodextrin, sulfobutyl ether cyclodextrin, dimethyl-cyclodextrin, and methyl-cyclodextrin.

In the present invention, examples of the cyclodextrin include, but are not limited to, α-cyclodextrin, β-cyclodextrin, γ-cyclodextrin, and derivatives and salts of these. Preferable examples of the cyclodextrin include β-cyclodextrin, and derivatives and salts thereof, and more preferable examples include hydroxypropyl-β-cyclodextrin and maltosyl-β-cyclodextrin. As the salts of the cyclodextrin and the derivatives thereof, pharmacologically acceptable salts are preferred, and examples include a salt with an inorganic base, a salt with an organic base, a salt with an inorganic acid, a salt with an organic acid, and a salt with a basic or acidic amino acid.

Suitable examples of the salt with an inorganic base include alkali metal salts such as a sodium salt and a potassium salt; alkali earth metal salts such as a calcium salt and a magnesium salt; and an aluminum salt and an ammonium salt.

Suitable examples of the salt with an organic base include salts with trimethylamine, triethylamine, pyridine, picoline, ethanolamine, diethanolamine, triethanolamine, tromethamine [tris(hydroxymethy)methylamine], tert-butylamine, cyclohexylamine, benzylamine, dicyclohexylamine, and N,N-dibenzylethylenediamine.

Suitable examples of the salt with an inorganic acid include salts with hydrochloric acid, hydrobromic acid, nitric acid, sulfuric acid and phosphoric acid.

Suitable examples of the salt with an organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, and p-toluenesulfonic acid.

Suitable examples of the salt with a basic amino acid include salts with arginine, lysine and ornithine.

Suitable examples of the salt with an acidic amino acid include salts with aspartic acid and glutamic acid.

The surfactant of the present invention is not especially limited, and preferable examples include a nonionic surfactant and a lecithin. Preferable examples of the nonionic surfactant include polyoxyethylene (10) octylphenyl ether (Triton (TM) X100), polysorbate 80, polysorbate 20, poloxamer 188 and N-dodecyl-B-D-maltoside, glycerin fatty acid ester, sucrose fatty acid ester, alkyl polyethylene glycol, alkyl glucoside, and polyoxyethylene polyoxypropylene glycol, and more preferable examples include Triton (TM) X100, polysorbate 80, polysorbate 20, poloxamer 188 and N-dodecyl-B-D-maltoside. Preferable examples of the lecithin include a soy lecithin and an egg yolk lecithin, and a more preferable example includes an egg yolk lecithin.

In the present invention, the content of the non-reducing sugar is preferably 0.1 to 94.99% by weight, more preferably 10 to 94.99% by weight, and further preferably 30 to 94.99% by weight of the entire microspike. In the present invention, the content of the sugar alcohol is preferably 0.1 to 94.99% by weight, more preferably 10 to 94.99% by weight and further preferably 30 to 94.99% by weight of the entire microspike. In the present invention, the content of the cyclodextrin is preferably 0.1 to 94.99% by weight, more preferably 10 to 94.99% by weight and further preferably 30 to 94.99% by weight of the entire microspike. In the present invention, the content of the surfactant is preferably 0.01 to 50% by weight, more preferably 0.01 to 30% by weight and further preferably 0.01 to 15% by weight of the entire microspike. Besides, in employing a combination of a non-reducing sugar, a sugar alcohol and a surfactant, the content of the combination is preferably 0.01 to 94.99% by weight, more preferably 10 to 94.99% by weight and further preferably 30 to 94.99% by weight of the entire microspike. Alternatively, in employing a combination of a non-reducing sugar and a sugar alcohol, the content of the combination is preferably 0.1 to 94.99% by weight, more preferably 10 to 94.99% by weight and further preferably 30 to 94.99% by weight of the entire microspike.

The amount of at least one selected from the group consisting of a non-reducing sugar, a sugar alcohol, a cyclodextrin and a surfactant is 0.01 to 94.99% by weight, more preferably 10 to 94.99% by weight and further preferably 30 to 94.99% by weight of the entire soluble microspike.

The ionic polymer base material of the present invention is not especially limited, and a preferable example includes at least one selected from the group consisting of polysaccharides, copolymers thereof and salts thereof. A copolymer herein refers to a copolymer of polysaccharides mentioned below, and is a polymer (a random copolymer, an alternating copolymer, a block copolymer or a graft copolymer) using two polysaccharides, such as a chitin-chitosan copolymer. As the polysaccharides, the copolymers thereof and the salts thereof, pharmacologically acceptable salts are preferably used, and examples include the same as those described above with respect to the cyclodextrin.

The ionic polymer base material is preferably a polysaccharide, and a preferable example of the polysaccharide includes at least one selected from the group consisting of chondroitin sulfuric acid, hyaluronic acid, chitosan and chitin, and salts of these. More preferable examples of the polysaccharide include sodium chondroitin sulfate, chitosan glutamate, chitosan hydrochloride, chitosan acetate, chitosan lactate, chitosan alginate and chitosan ascorbate. More preferable examples include sodium chondroitin sulfate and chitosan glutamate. In the present invention, the content of the ionic polymer base material is preferably 1 to 99.98% by weight, more preferably 1 to 70% by weight and further preferably 1 to 50% by weight of the entire microspike.

Assuming that the amount (weight) of the ionic polymer base material mixed is 1, the amount (weight) of the non-reducing sugar, the sugar alcohol, the cyclodextrin or the surfactant mixed is preferably 0.1 to 99, more preferably 0.1 to 70, and further preferably 0.1 to 50.

In the present invention, as the vaccine antigen, for example, a toxoid and a vaccine antigen having a particulate structure can be used, and a single one of these or a mixture of these can be used. Examples of the toxoid include, but are not limited to, a tetanus toxoid and a diphtheria toxoid. Examples of the vaccine antigen having a particulate structure include, but are not limited to, virus vaccines, such as a norovirus vaccine, a Dengue fever vaccine, an HPV vaccine, an influenza vaccine, and a rotavirus vaccine. Preferable examples of the virus vaccines include a norovirus vaccine and a rotavirus vaccine, and a more preferable example includes a norovirus vaccine. In the present invention, the content of the vaccine antigen is preferably 0.01 to 10% by weight, more preferably 0.01 to 8% by weight and further preferably 0.01 to 6% by weight of the entire microspike.

In the present invention, the microspike may further contain an adjuvant. Examples of the adjuvant include adjuvants usually used in producing vaccine preparations, such as a water-insoluble adjuvant, a hydrophilic gel adjuvant and a water-soluble adjuvant.

Examples of the water-insoluble adjuvant include a retinoid such as retinoic acid, 4-amino-1-(2-methylpropyl)-1H-imidazo[4,5-c]quinoline (imiquimod), imidazoquinolines such as 1-[4-amino-2-(ethoxymethyl)imidazo[4,5-c]quinolin-1-yl]-2-methylpropan-2-ol (Resquimod (R-848)), 4-amino-α,α,2-dimethyl-1H-imidazo[4,5-c]quinoline-1-ethanol (R-842 (manufactured by 3M Pharmaceuticals, or the like); see Journal of Leukocyte Biology (1995) 58: 365-372), 4-amino-α,α,2-trimethyl-1H-imidazo[4,5-c]quinoline-1-ethanol (S-27609) (manufactured by 3M Pharmaceuticals or the like); see Journal of Leukocyte Biology(1995) 58: 365-372), and 4-amino-2-ethoxymethyl-α,α-dimethyl-1H-imidazo[4,5-c]quinoline-1-ethanol (S-28463 (manufactured by 3M Pharmaceuticals or the like); see Antivirul Research (1995) 28: 253-264), Loxoribine, Bropirimine, oleic acid, liquid paraffin, and Freund. Examples of the hydrophilic gel adjuvant include aluminum hydroxide and aluminum phosphate. Examples of the water-soluble adjuvant include α-defensin, β-defensin, cathelicidin, sodium alginate, poly[di(carboxylatophenoxy)phosphazene], saponin extract (Quil A) and polyethyleneimine.

The content of the adjuvant is 0 to 1500% by weight, more preferably 0 to 1000% by weight and further preferably 0 to 500% by weight based on the vaccine antigen.

Combinations according to preferable aspects of the present invention are as follows:
(1) A combination of at least one selected from the group consisting of a non-reducing sugar, a sugar alcohol, a cyclodextrin and a surfactant, a vaccine antigen and an ionic polymer base material.
(2) A combination of a non-reducing sugar, a vaccine antigen and an ionic polymer base material.
(3) A combination of a sugar alcohol, a vaccine antigen and an ionic polymer base material.
(4) A combination of a cyclodextrin, a vaccine antigen and an ionic polymer base material.
(5) A combination of a surfactant, a vaccine antigen and an ionic polymer base material.
(6) A combination of an oligosaccharide as a non-reducing sugar, a tetanus toxoid, a diphtheria toxoid, norovirus or rotavirus as a vaccine antigen, and a polysaccharide.
(7) A combination of trehalose or sucrose as a non-reducing sugar, a tetanus toxoid, a diphtheria toxoid or norovirus as a vaccine antigen, and sodium chondroitin sulfate or chitosan glutamate as a polysaccharide.
(8) A combination of mannitol, sorbitol, glycerol, xylitol or erythritol as a sugar alcohol, a tetanus toxoid, a diphtheria toxoid, norovirus or rotavirus as a vaccine antigen, and a polysaccharide as an ionic polymer base material.
(9) A combination of mannitol, sorbitol or glycerol as a sugar alcohol, a tetanus toxoid, a diphtheria toxoid or norovirus as a vaccine antigen, and sodium chondroitin sulfate or chitosan glutamate as an ionic polymer base material.
(10) A combination of β-cyclodextrin, a derivative thereof and a salt thereof as a cyclodextrin, a tetanus toxoid, a diphtheria toxoid, norovirus or rotavirus as a vaccine antigen, and a polysaccharide as an ionic polymer base material.
(11) A combination of hydroxypropyl-β-cyclodextrin or maltosyl-β-cyclodextrin as a cyclodextrin, a tetanus toxoid, a diphtheria toxoid or norovirus as a vaccine antigen, and sodium chondroitin sulfate or chitosan glutamate as an ionic polymer base material.
(12) A combination of a nonionic surfactant or a lecithin as a surfactant, a tetanus toxoid, a diphtheria toxoid, norovirus or rotavirus as a vaccine antigen, and a polysaccharide as an ionic polymer base material.
(13) A combination of Triton (TM) X100, polysorbate 80, polysorbate 20, poloxamer 188 and N-dodecyl-B-D-maltoside, or an egg yolk lecithin as a surfactant, a tetanus toxoid, a diphtheria toxoid or norovirus as a vaccine antigen, and sodium chondroitin sulfate or chitosan glutamate as an ionic polymer base material.
(14) A combination of trehalose or sucrose as a non-reducing sugar, norovirus as a vaccine antigen, and sodium chondroitin sulfate or chitosan glutamate as a polysaccharide.
(15) A combination of mannitol or sorbitol as a sugar alcohol, norovirus as a vaccine antigen, sodium chondroitin sulfate or chitosan glutamate as a polysaccharide.

In the preparation according to one aspect of the present invention, the projection may directly be held on the support unified with the base. The support may be in the shape of a sheet or a plate, and may be or may not be an adhesive sheet. As the support, for example, a commercially available double sided adhesive tape can be used. As for a material of the double sided adhesive tape, one obtained by applying a pressure sensitive adhesive material used for a medical tape, such as an acrylic pressure sensitive adhesive or a silicone pressure sensitive adhesive, onto both sides of a support of a polyester film or nonwoven fabric can be used.

In another aspect of the preparation of the present invention, the preparation can be prepared by forming, by using the base, the spike holding member for holding the projection, and by holding the spike holding member on the support.

The projection may be in any shape suitable for sticking in a body surface, and the shape includes a cylindrical shape, is preferably a shape tapered from a large base toward a thin tip, and may be a needle shape, a pyramid shape, a conical shape, or any polygonal pyramid shape such as a triangular pyramid, a square pyramid, a pentagonal pyramid, a hexagonal pyramid, a heptagonal pyramid, an octagonal pyramid, a nonagonal pyramid, a decagonal pyramid, a hendecagonal pyramid, a dodecagonal pyramid, or another polygonal pyramid.

The length (the height) of the projection is preferably 10 to 1000 µm, preferably 100 to 800 µm, and preferably 100 to 600 µm. If the projection is in the shape of a cylinder, the base diameter is preferably 10 to 500 µm, preferably 100 to 500 µm, and preferably 100 to 400 µm, and the tip diameter of the projection is preferably 0.1 to 20 µm, preferably 0.1 to 10 µm, and preferably 0.1 to 5 µm. If the projection is in the shape of a pyramid, the length of one side at the base is preferably 10 to 500 µm, preferably 100 to 500 µm, and preferably 100 to 400 µm, and the length of one side at the tip of the projection is preferably 0.1 to 20 µm, preferably 0.1 to 10 µm, and preferably 0.1 to 5 µm. The entire projection can be a microspike containing the vaccine antigen.

The projection may include a plurality of layers, consisting of a layer of the microspike containing the vaccine antigen and a layer not containing the vaccine antigen, arranged in either a parallel direction or a vertical direction to the base, and the layers are preferably in parallel to the base. If the plural layers are in parallel to the base, the microspike may correspond to a tip layer of the projection, or an intermediate layer. Besides, the respective layers may have different compositions. If the microspike corresponds to the tip layer of the projection, the length of the microspike from the tip is preferably 0.01 to 800 µm, preferably 0.01 to 500 µm, and preferably 0.01 to 300 µm. If the microspike corresponds to the intermediate layer of the projection, the distance of the microspike from its bottom is preferably 50 to 999 µm, preferably 50 to 500 µm, and preferably 50 to 300 µm.

The preparation of the present invention is suitably in a rectangular or circular shape, but may be in another shape as long as the object of the present invention can be attained.

As for the size of the preparation of the present invention, the preparation can advantageously be handled, if it is in a rectangular shape, by setting one side to about 1 mm to about 50 mm, preferably about 5 mm to 30 mm, more preferably about 10 mm to 20 mm, and if it is in a circular shape, by setting the diameter to about 1 mm to about 50 mm, preferably about 5 mm to 30 mm, and more preferably about 10 mm to 20 mm.

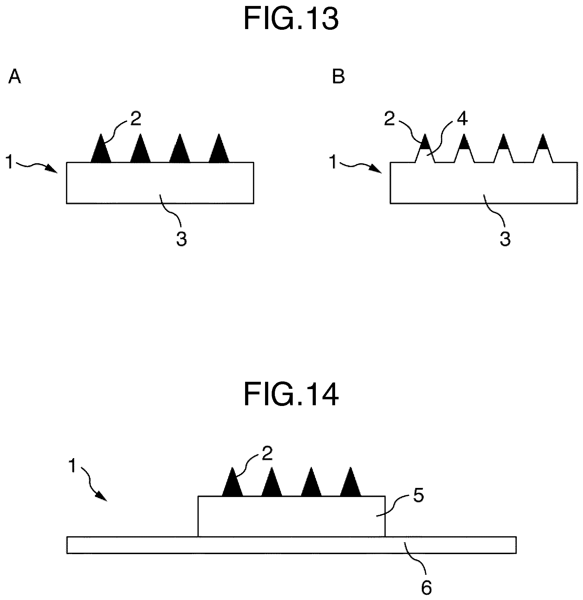

Figs. 13A and 13B are conceptual diagrams of examples of the preparation of the present invention.

In a preparation 1, the entire projection may correspond to the microspike containing the vaccine antigen, and Fig. 13A illustrates a conceptual diagram of an example of the preparation 1 in which a microspike 2 corresponds to the entire projection. In this example, the microspike 2 is in a shape projecting from a support 3 unified with a base. The microspike 2 is directly bonded to the support 3 unified with the base.

Fig. 13B illustrates a conceptual diagram of an example of the preparation 1 in which the projection includes, in parallel to the base, a layer of the microspike containing the vaccine antigen and a layer not containing the vaccine antigen, and the microspike 2 corresponds to a tip layer of the projection. Also in this example, the microspike 2 containing the vaccine antigen is in a shape projecting from the support 3 unified with the base. On the other hand, the microspike 2 is bonded, via a projection base 4 not containing the vaccine antigen, to the support 3 unified with the base.

Fig. 14 illustrates a conceptual diagram of still another example of the preparation of the present invention. In this example, the microspike 2 is directly bonded to the base 5 working as the spike holding member and is in a shape projecting from the base 5. The base 5 is held on the support 6. Specifically, the preparation 1 is constituted by holding the microspike 2 on the support 6 with the base 5 sandwiched therebetween.

The preparation of the present invention can be molded by using a shaping mold or another known technique. The shaping mold is provided with a hole or a recess for forming the projection. The shaping mold may be made of, but is not limited to, a metal, a ceramic, or a polymer such as a rubber, a resin, a silicon resin or a fluororesin. The shaping mold can be formed by pressing a plastic material against a mold provided with a convex portion having the shape of the projection and subsequently dissociating the material from the mold. In this case, the plastic material may be, but is not limited to, for example, a thermoplastic polymer such as a thermoplastic rubber, resin, silicon resin or fluororesin, and also encompasses a styrene-based elastomer. Besides, the mold provided with a convex portion is not limited but may be, for example, a mold of metal. As an unrestricted example, the shaping mold can be obtained by placing a thermoplastic polymer over a heated mold followed by pressing, cooling the thermoplastic polymer and the mold, and then dissociating the thermoplastic polymer from the mold. In this case, the thermoplastic polymer may be or may not be in a sheet shape.

If the shaping mold is used, the preparation of the present invention may be produced through the following process.

The respective components of the microspike are mixed with water, another solvent or a mixed solution of water and another solvent to obtain a mixture, and the mixture is poured into the hole or the recess of the shaping mold. The poured mixture is preferably filled in the hole or the recess. Means for filling it is not limited but may be, for example, air press or centrifugation. Examples of the solvent used here include ethanol, methanol, acetonitrile, acetone, dichloromethane and chloroform.

The production of the preparation of the present invention (such as the preparation illustrated in Fig. 13A or 13B) including the support unified with the base is not especially limited, and for example, the following method may be employed for the production. Specifically, after a mixture containing the respective components of the microspike is filled in the hole or the recess of the shaping mold, the support unified with the base is placed over the shaping mold, and then, the support is dissociated from the shaping mold to collect a projection held thereon. The support unified with the base may be in the shape of a sheet, a tape, a plate, a block or another shape, and may be or may not be adhesive. As the support unified with the base, those described above as usable as the support can be used. The support unified with the base and holding the projection can directly be used as the preparation.

If the support is a member separate from the base and the base works as the spike holding member, the production of the preparation of the present invention (such as the preparation illustrated in Fig. 14) is not especially limited, and for example, the following method may be employed for the production. Specifically, after a mixture containing the respective components of the microspike is filled in the hole or the recess of the shaping mold, the base working as the spike holding member is placed over the shaping mold, and then the base is dissociated from the shaping mold to collect a projection held thereon. In this case, the base may be in the shape of, for example, but is not limited to, a sheet, a tape, a plate, a block or another shape, and may be or may not be adhesive, and also encompasses a double sided adhesive tape. The preparation is produced by causing the spike holding member, that is, the base holding the projection, to adhere to the support. The base material of the base working as the spike holding member may be a solidifying material, and is not limited, but for example, the ionic polymer base material of the present invention, a resin sheet of polyvinylchloride, silicone rubber, a thermoplastic elastomer, polypropylene, polyethylene, polyethylene terephthalate, polycarbonate, polystyrene, polytetrafluoroethylene or polyurethane, or flexible paper, a nonwoven fabric, a fabric, a foam or a metal can be used.

In either aspect, drying or solidifying is performed for forming the projection, and the drying or solidifying may be performed before or after taking the projection out of the shaping mold.

In another aspect of the production method for the preparation of the present invention, a base material not containing a vaccine antigen may be first poured into the hole or the recess of the shaping mold, and thereafter respective components of the microspike containing the vaccine antigen may be poured into the hole or the recess of the shaping mold. In this aspect, a projection including a plurality of layers in which a tip layer does not contain the vaccine antigen but an intermediate layer contains the vaccine antigen can be formed. In still another aspect of the production method for the preparation of the present invention, in order to cause a projection to include a plurality of layers having different component concentrations, mixtures in accordance with the component concentrations may successively be poured into the hole or the recess of the shaping mold.

As the base material not containing the vaccine antigen, at least one selected from the group consisting of the ionic polymer base material of the present invention, a nonionic polymer, an acrylic acid-based polymer and a methacrylic acid-based polymer is used, and the ionic polymer base material of the present invention, an acrylic acid-based polymer or a methacrylic acid-based polymer is preferably used. More preferably, the ionic polymer base material of the present invention is used, and an example includes at least one selected from the group consisting of polysaccharides, copolymers of these and salts of these. A more preferable example includes a polysaccharide, and an example of the polysaccharide includes at least one selected from the group consisting of chondroitin sulfuric acid, hyaluronic acid, chitosan and chitin, and salts of these. More preferable examples of the polysaccharide include sodium chondroitin sulfate, chitosan glutamate, chitosan hydrochloride, chitosan acetate, chitosan lactate, chitosan alginate and chitosan ascorbate. Still more preferable examples include sodium chondroitin sulfate and chitosan glutamate.

The nonionic polymer refers to polyvinyl pyrrolidone, polyvinyl alcohol, polyethylene glycol, polyethylene oxide, polyacrylamide, dextran, polylactic acid, polyglycolic acid or a lactic acid/glycolic acid copolymer.

The acrylic acid-based polymer refers to a carboxyvinyl polymer, polyacrylic acid, sodium polyacrylate, or a copolymer of acrylic acid/sodium acrylate.

The methacrylic acid-based polymer refers to a methacrylic acid copolymer or an aminoalkyl methacrylate copolymer.

In another aspect, if the preparation has the projection base, as a base material of the projection base, at least one selected from the group consisting of the ionic polymer base material of the present invention, a nonionic polymer base material, an acrylic acid-based polymer and a methacrylic acid-based polymer is used. Here, examples of the nonionic polymer base material, the acrylic acid-based polymer and the methacrylic acid-based polymer include those mentioned above. As the base material of the projection base, the ionic polymer base material of the present invention, the acrylic acid-based polymer or the methacrylic acid-based polymer is preferably used. More preferably, the ionic polymer base material of the present invention is used, and an example includes at least one selected from the group consisting of polysaccharides, copolymers of these and salts of these. A more preferable example includes a polysaccharide, and a preferable example of the polysaccharide includes at least one selected from the group consisting of chondroitin sulfuric acid, hyaluronic acid, chitosan and chitin, and salts of these. More preferable examples of the polysaccharide include sodium chondroitin sulfate, chitosan glutamate, chitosan hydrochloride, chitosan acetate, chitosan lactate, chitosan alginate and chitosan ascorbate. Still more preferable examples include sodium chondroitin sulfate and chitosan glutamate.

The preparation of the present invention can be applied to a mammal (such as a human, a monkey, sheep, a horse, a dog, a cat, a rabbit, a rat or a mouse) for purpose of treatment, prevention and the like with the drug.

As a method for using the preparation of the present invention, the preparation can be applied to any position of the skin, and can be used also in an uneven region.

A dose of the drug by using the preparation of the present invention is varied depending on the degree of symptom, the age, sex and weight of an administration target, the period and interval of the administration, the type of an active ingredient, and the like, and may be selected from a range where the dose as a pharmaceutical active ingredient can be an effective dose. Besides, the preparation of the present invention may be administered in one dose or two or three divided doses per day.

The preparation of the present invention is useful for the treatment, the prevention and the like with the drug.

The preparation of the present invention can contain the vaccine antigen in an amount necessary for the treatment and the prevention.

A target disease and an amount of the drug necessary for the disease are described, for example, in Japan, in Minimum Requirements for Biological Products notified by the Ministry of Health and Welfare, and in equivalent official specifications or the like in the other countries. The amount of the drug to be administered cannot uniformly be defined in accordance with, for example, the purpose of inoculating the vaccine (such as initial inoculation, additional inoculation or the like), whether or not it is a combined vaccine, the age of a patient to be inoculated, the manufacturer, the virus strain and type, and therefore, generally used drug amounts are herein exemplarily described, but it is noted that the application to the present invention is not limited to the described amounts. The generally used drug amounts are, for example, 2.5 to 5 Lf for tetanus, 15 to 25 Lf for diphtheria, 20 to 40 micrograms for each type of human papilloma virus, 10⁶ CCID₅₀ or more for rotavirus, 5 to 500 micrograms for norovirus, 10³ PFU or more and 10¹⁰ PFU or less for Dengue fever, and 15 micrograms or more and 100 micrograms or less (in HA content) for influenza.

The preparation of the present invention can be used together with another preparation, such as an oral administration preparation or an injection.

In the present invention, if the ionic polymer is used as the base material, an effect of retaining or improving the strength necessary as a needle of a microneedle is exhibited. Besides, in accordance with a mixing ratio between the ionic polymer base material of the present invention and a non-reducing sugar, a sugar alcohol, a cyclodextrin or a surfactant, an effect of suppressing degradation, aggregation or the like of the antigen otherwise caused during drying or storage to improve the biological stability is exhibited. In general, if a vaccine antigen is naturally dried under an environment of room temperature or air dried, it is difficult to retain the activity persistence, and therefore, freeze-drying, spray-drying or the like is generally used as a method for drying a vaccine antigen. The term "drying" used herein refers to, however, drying under a severe environment, such as natural drying under an environment of room temperature and reduced pressure over 18 hours, natural drying under an environment of room temperature and normal pressure over 18 hours, or air drying under an environment of room temperature and normal pressure over several minutes to several hours (for example, 1 minute to 2 hours, or 1 minute to 1 hour), and even under these environments, the degradation of the antigen can be suppressed by the present invention. Here, room temperature refers to a temperature range of 15°C to 35°C.

Besides, it is generally difficult to retain the activity persistence of a vaccine antigen during storage under a temperature environment of room temperature or higher, and hence, a vaccine antigen is generally stored under a refrigerated or frozen environment. The term "during storage" herein refers to, however, storage under a severe environment, such as storage under an environment of 25°C and 65%RH or 40°C and 75%RH, and even under these environments, the degradation of the antigen can be suppressed by the present invention. Furthermore, in accordance with the mixing ratio between the ionic polymer base material of the present invention and a non-reducing sugar, a sugar alcohol, a cyclodextrin or a surfactant, effects of providing the microspike with sufficient strength to be used for inserting into a body surface, and of biologically stabilizing the vaccine antigen in a solid state are exhibited. For example, the strength of the soluble microspike can be measured by using a micro compression testing machine, a needle tip strength testing machine, a micro strength evaluation tester or the like, and the stability of the antigen in a solid state can be measured by size exclusion chromatography, reverse phase chromatography, electrophoresis, particle size measurement, a CD spectrum or the like. The strength of the present invention corresponds, if it is measured by using, for example, a needle tip strength testing machine (manufactured by ASTI Corporation), to a range where a measurement value of 5 to 300 gf is obtained as a pressure corresponding to a moving distance of 0.1 mm, and a preparation having strength of 10 to 200 gf, and further 20 to 100 gf can be produced. According to the present invention, a preparation having proper and necessary strength (over the weaker range to the stronger range described above: 5 to 300 gf) in accordance with the drug or the like for intradermal administration can be produced. The stability attained by the present invention corresponds, if it is measured by, for example, the reverse phase chromatography, to a range where the degradation or the aggregation of the antigen is suppressed, in the entire antigen, to 0 to 30%, and includes a range where it is suppressed further to 0 to 15%, and still further to 0 to 10%. Alternatively, if it is measured by, for example, the size exclusion chromatography, the stability corresponds to a range where the degradation or the aggregation of the antigen is suppressed, in the entire antigen, to 0 to 35%, and includes a range where it is suppressed further to 0 to 20%, and still further to 0 to 10%. Moreover, owing to the component structure of the microspike of the present invention, an effect of making the microspike soluble in vivo is exhibited. Specifically, according to the present invention, the "stabilization" can be attained in terms of stabilization against chemical change and stabilization against physical change, and particularly, an effect of stabilization for the vaccine antigen retaining the antigenic activity and stabilization for attaining the strength as a spike of the preparation can be exhibited.

It is noted that a microneedle in which a drug is coated on the surface of a refined needle, namely, a drug-coated microneedle, is not implied in the preparation of the present invention. In other words, even if a drug has solubility in a drug layer coated on the surface of a needle in a drug-coated microneedle, the refined needle is not encompassed by the soluble microspike of the present invention.

The preparation of the present invention can be used for preventing or treating a disease by administering the vaccine antigen. Examples of the disease include infectious diseases such as tetanus, diphtheria, norovirus, Dengue fever, human papilloma virus (HPV), influenza and rotavirus, and other diseases that are prevented or treated by using vaccine antigens.

The preparation of the present invention is stabilized as described above, and hence is safe, has no toxicity and is useful for the treatment, prevention and the like.

The present invention will now be described in more detail on the basis of examples, but it is noted that the present invention is not limited to these examples. With respect to reagents and the like to be used, products compliant with Japanese Pharmacopoeia and Japanese Pharmaceutical Excipients were appropriately used.

### Examples

### Production of Microneedle

### (Example 1)

After 30 mg of ovalbumin (manufactured by Wako Pure Chemical Industries, Ltd.), 90 mg of chitosan glutamate (PROTASAN (Registered Trademark) UP G213, manufactured by FMC BioPolymer) and 180 mg of sucrose (manufactured by Wako Pure Chemical Industries, Ltd.) were added to and dissolved in 5700 µL of purified water, bubbles present in the resultant solution were removed under vacuum to obtain a drug filling solution.

A microneedle mold (needle material: SUS316L, needle shape: square pyramid, side length: 300 µm, height: 500 µm, arrangement: 1 mm pitch x 10 columns x 10 rows = 100 needles, square shape, manufactured by Tokai Azumi Techno Co., Ltd.) was heated at 179°C on a heating plate of a mold making tool.

A sheet of a styrene-based thermoplastic elastomer (RABARON (Registered Trademark), with a thickness of 1 mm, manufactured by Mitsubishi Chemical Corporation) was cut into a size of about 2.5 cm x 2.5 cm, and the cut sheet was placed over the heated mold and pressed for 30 seconds at a press pressure of about 25 N. The resultant sheet and mold were cooled at room temperature for about 1 minute, and then the sheet was peeled off from the mold to obtain a microneedle shaping mold having recesses each in a square pyramid shape.

The shaping mold was set on an XY stage of a microneedle manufacturing apparatus, and a dispenser (nozzle size: 0.075 mm in diameter) attached to the manufacturing apparatus was used to discharge the drug filling solution into the recesses of the shaping mold (the arrangement of recesses: 1 mm pitch x 10 columns x 10 rows = 100 recesses). After discharging, the air press was performed for 60 seconds by using a pneumatic press for filling the drug filling solution into the innermost portion of each recess. Thereafter, the shaping mold was dried at room temperature for about 18 hours, and then an acrylic surface of a double sided adhesive tape (No. 5302A, manufactured by Nitto Denko Corporation) was applied onto a surface of the shaping mold and was peeled, so as to collect microneedles on a tape adhesive surface. The thus collected microneedles were caused to adhere onto a surface of a soft polyethylene sheet having a length of 18 mm and a thickness of 0.3 mm via the double sided adhesive tape, and thus, a microneedle patch holding the 100 microneedles thereon was obtained.

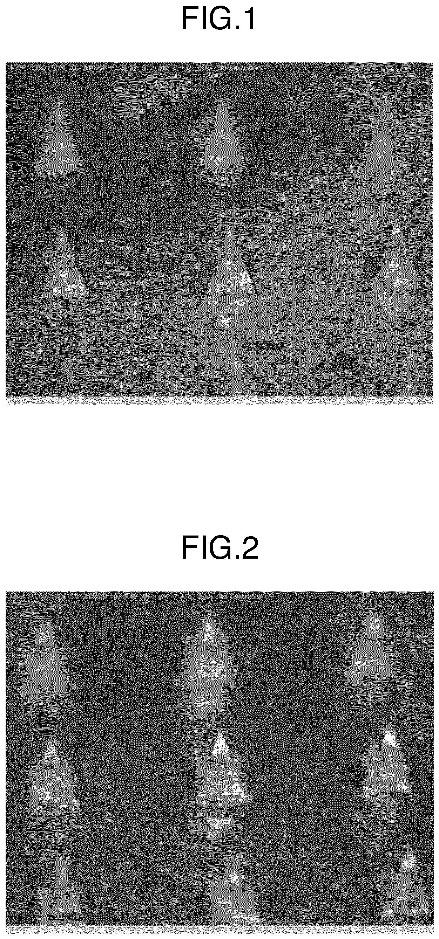

A micrograph of the thus obtained microneedle patch is illustrated in Fig. 1. Each microneedle was in a square pyramid shape having a base length of 300 µm and a height of 500 µm, which was the same shape as that of the used mold. The content of ovalbumin per microneedle patch was 35 µg.

### (Example 2)

After 30 mg of ovalbumin (manufactured by Wako Pure Chemical Industries, Ltd.), 30 mg of chitosan glutamate (PROTASAN (Registered Trademark) UP G213, manufactured by FMC BioPolymer) and 240 mg of sucrose (manufactured by Wako Pure Chemical Industries, Ltd.) were added to and dissolved in 1700 µL of purified water, bubbles present in the resultant solution were removed under vacuum to obtain a drug filling solution.

A microneedle shaping mold obtained in the same manner as in Example 1 was used to obtain a microneedle patch by a similar method to Example 1.

A micrograph of the thus obtained microneedle patch is illustrated in Fig. 2. Each microneedle was in a square pyramid shape having a base length of 300 µm and a height of 500 µm, which was the same shape as that of the used mold. The content of ovalbumin per microneedle patch was 172 µg.

### (Example 3)

After 15 mg of ovalbumin (manufactured by Wako Pure Chemical Industries, Ltd.), 15 mg of chitosan glutamate (PROTASAN (Registered Trademark) UP G213, manufactured by FMC BioPolymer), 270 mg of sucrose (manufactured by Wako Pure Chemical Industries, Ltd.) and 0.1 mg of Acid Red 52 (manufactured by Wako Pure Chemical Industries, Ltd.) were added to and dissolved in 700 µL of purified water, bubbles present in the resultant solution were removed under vacuum to obtain a drug filling solution.

A microneedle shaping mold obtained in the same manner as in Example 1 was used to obtain a microneedle patch by a similar method to Example 1.

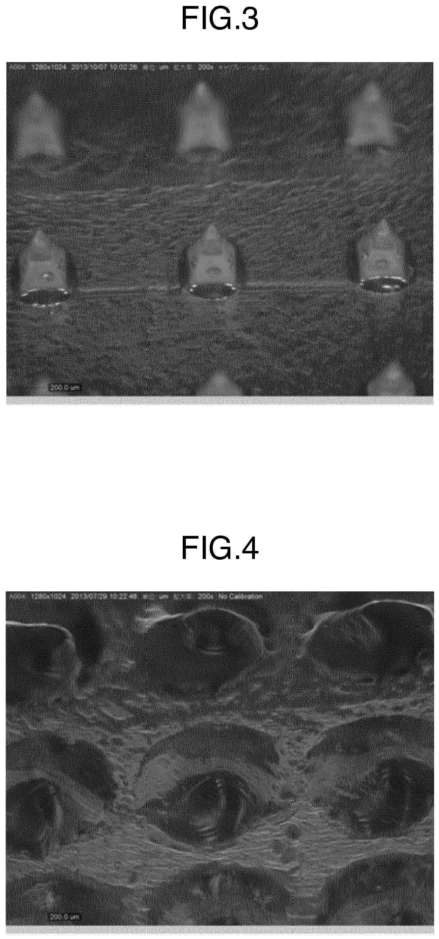

A micrograph of the thus obtained microneedle patch is illustrated in Fig. 3. Each microneedle was in a square pyramid shape having a base length of 300 µm and a height of 500 µm, which was the same shape as that of the used mold. The content of ovalbumin per microneedle patch was 103 µg.

### (Comparative Example 1)

After 90 mg of ovalbumin (manufactured by Wako Pure Chemical Industries, Ltd.), 810 mg of sucrose (manufactured by Wako Pure Chemical Industries, Ltd.) and 0.3 mg of Evans Blue (manufactured by Wako Pure Chemical Industries, Ltd.) were added to and dissolved in 600 µL of purified water, bubbles present in the resultant solution were removed under vacuum to obtain a drug filling solution.

A microneedle shaping mold obtained in the same manner as in Example 1 was used to collect a solid content on a tape adhesive surface by a similar method to Example 1. The thus collected solid content was caused to adhere to a surface of a soft polyethylene sheet having a length of 18 mm and a thickness of 0.3 mm via the double sided adhesive tape, so as to obtain a patch holding 100 lumps of the solid content thereon.

A micrograph of the thus obtained patch is illustrated in Fig. 4. Although the solid content was found on the surface of the patch, no needle was formed.

The compositions and moldability of the microneedles of Examples 1 to 3 and Comparative Example are shown in Table 1. In the formulation containing, as the base material, 5% by weight or more chitosan glutamate in addition to sucrose, needles in the same shape as the mold were formed, whereas in the formulation not containing chitosan glutamate, no needle was formed.

### [Table 1]

**Table 1**

| Composition and Moldability of Microneedle | | | |
|---|---|---|---|
| | Components | Weight Ratio | Moldability |
| Example 1 | Ovalbumin, | 10 | Needle in the same shape as mold formed |
| | Chitosan Glutamate, | 30 | |
| | Sucrose | 60 | |
| Example 2 | Ovalbumin, | 10 | Needle in the same shape as mold formed |
| | Chitosan Glutamate, | 10 | |
| | Sucrose | 80 | |
| Example 3 | Ovalbumin, | 5 | Needle in the same shape as mold formed |
| | Chitosan Glutamate, | 5 | |
| | Sucrose, | 90 | |
| | Acid Red 52 | 0.03 | |
| Comparative Example 1 | Ovalbumin, | 10 | No needle formed |
| | Sucrose, | 90 | |
| | Evans Blue | 0.03 | |

Stability Test of Vaccine Antigen

### (Example 4)

### Sample Preparation:

A solution was prepared by mixing and dissolving 1 ml of a tetanus toxoid solution (9.7 mg/ml) (T) and 97 mg of sodium chondroitin sulfate (CS). To the thus obtained tetanus toxoid-CS mixed solution, 0.97 mg of Triton X100 was added and well mixed, and the resultant was dividedly poured into two plastic tubes in an amount of 2.1 µl each. One of the tubes was used as an initial sample, and the other was air dried and then sealed in an aluminum pouch to be stored under conditions of 40°C and 75%RH for 1 week. Similarly, solutions respectively containing other additives (polysorbate 80, polysorbate 20, poloxamer 188, N-dodecyl-B-D-maltoside, and a lecithin) were also prepared to have mixing amounts shown in Table 2, so as to prepare initial samples and samples stored at 40°C.

### Evaluation:

(1) To each of the plastic tubes of the initial samples, 5 µl of NUPAGE LDS Sample buffer (4X) (Life Technologies), 2 µl of NUPAGE Reducing Agent (10X)
(Life technology) and 10.9 µl of water were added and mixed, and the resultant was heated at 90°C for 5 minutes.

Each of the samples after stored at 40°C for 1 week was dissolved in 10 µl of water, and then a protein concentration was quantitatively determined by using NANODROP 2000C (manufactured by Thermo Scientific). The sample was collected to have a protein content of 4 µg, 5 µl of NUPAGE LDS Sample buffer (4X) and 2 µl of NUPAGE Reducing Agent (10X) were added thereto, and water was further added thereto to attain a total amount of 20 µl. All of these samples were heated at 90°C for 5 minutes.
(2) Each of the samples prepared as described in (1) above and a molecular weight maker Hi Mark™ Pre-Stained Protein Standard (Life Technologies) were added to NUPAGE Bis-Tris MiniGels (Life Technologies), and the resultant was subjected to the electrophoresis at 200 V for 50 minutes by using Powder Pac HC (manufactured by Bio-Rad). A gel obtained after the electrophoresis was stained with Simply Blue™ SafeStain (Life Technologies).

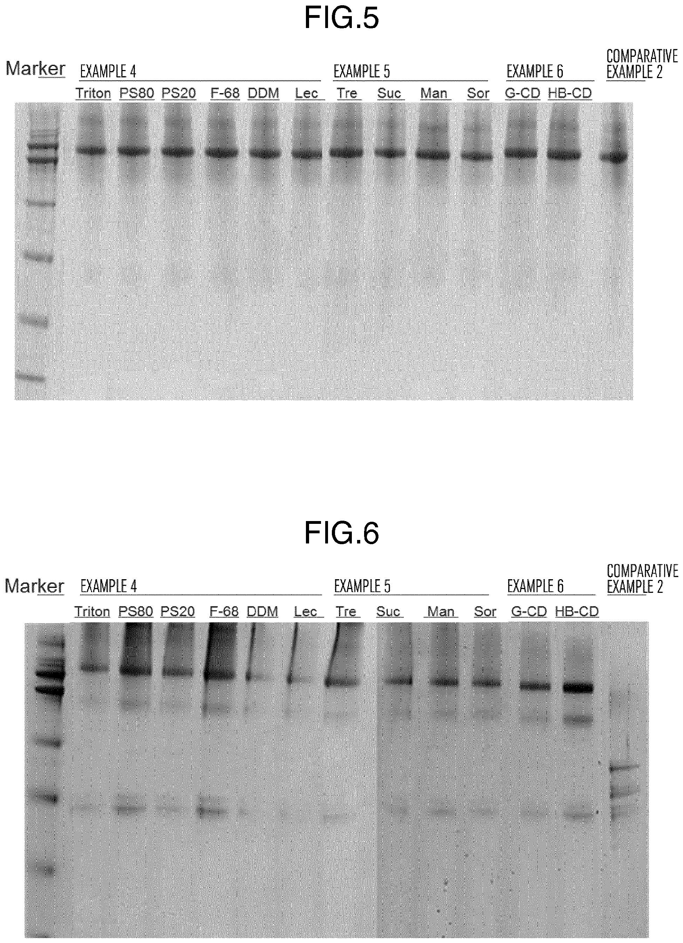

Results: Results are illustrated in Fig. 5 (initial samples) and Fig. 6 (samples after stored at 40°C for 1 week).

### (Example 5)

### Sample Preparation:

A solution was prepared by mixing and dissolving 1 ml of a tetanus toxoid solution (9.7 mg/ml) (T) and 97 mg of sodium chondroitin sulfate (CS). To the thus obtained tetanus toxoid-CS mixed solution, 48.5 mg of trehalose was added and well mixed, and the resultant was dividedly poured into two plastic tubes in an amount of 2.1 µl each. One of the tubes was used as an initial sample, and the other was air dried and then sealed in an aluminum pouch to be stored under conditions of 40°C and 75%RH for 1 week. Similarly, solutions respectively containing other additives (sucrose, mannitol and sorbitol) were also prepared to have mixing amounts shown in Table 2, so as to prepare initial samples and samples stored at 40°C.

Evaluation: The evaluation was performed by SDS-PAGE in the same manner as in Example 4.

Results: Results are illustrated in Fig. 5 (initial samples) and Fig. 6 (samples after stored at 40°C for 1 week).

### (Example 6)

### Sample Preparation:

A solution was prepared by mixing and dissolving 1 ml of a tetanus toxoid solution (9.7 mg/ml) (T) and 97 mg of sodium chondroitin sulfate (CS). To the thus obtained tetanus toxoid-CS mixed solution, 48.5 mg of G2-β-cyclodextrin was added and well mixed, and the resultant was dividedly poured into two plastic tubes in an amount of 2.1 µl each. One of the tubes was used as an initial sample, and the other was air dried and then sealed in an aluminum pouch to be stored under conditions of 40°C and 75%RH for 1 week. Similarly, a solution containing another additive (HP-β-cyclodextrin) was also prepared to have a mixing amount shown in Table 2, so as to prepare an initial sample and a sample stored at 40°C.

Evaluation: The evaluation was performed by the SDS-PAGE in the same manner as in Example 4.

Results: Results are illustrated in Fig. 5 (initial samples) and Fig. 6 (samples after stored at 40°C for 1 week).

### (Comparative Example 2)

### Sample Preparation:

One ml of a tetanus toxoid solution (9.7 mg/ml) was dividedly poured into two plastic tubes in an amount of 2.1 µl each. One of the tubes was used as an initial sample, and the other was air dried and then sealed in an aluminum pouch to be stored under conditions of 40°C and 75%RH for 1 week.

### Evaluation:

(1) To the plastic tube of the initial sample, 5 µl of NUPAGE LDS Sample buffer (4X) (Life Technologies), 2 µl of NUPAGE Reducing Agent (10X) (Life Technologies) and 10.9 µl of water were added and mixed, and the resultant was heated at 90°C for 5 minutes.

The sample after stored at 40°C for 1 week was dissolved in 10 µl of water, and then a protein concentration was quantitatively determined by using NANODROP 2000C (manufactured by Thermo Scientific). Since the protein content was not more than a detection limit, 5 µl of NUPAGE LDS Sample buffer (4X) and 2 µl of NUPAGE Reducing Agent (10X) were added to the total amount of the sample solution, and then water was further added thereto to attain a total amount of 20 µl. Thereafter, the resultant was heated at 90°C for 5 minutes.
(2) Each of the samples prepared as described in (1) above and a molecular weight marker Hi Mark™ Pre-Stained Protein Standard (Life Technologies) were added to NUPAGE Bis-Tris MiniGels (Life Technologies), and the resultant was subjected to the electrophoresis at 200 V for 50 minutes by using Powder Pac HC (manufactured by Bio-Rad). A gel obtained after the electrophoresis was stained with Simply Blue™ SafeStain (Life Technologies).

Results: Results are illustrated in Fig. 5 (initial sample) and Fig. 6 (sample after stored at 40°C for 1 week).

As illustrated in Figs. 5 and 6, the tetanus toxoid protein was more stable in the samples of Examples 4 to 6 than in the sample (T) of Comparative Example 2.

### [Table 2]

**Table 2**

| | | Example 4 | | | | | | Example 5 | | | | Example 6 | | Comparative Example 2 |
|---|---|---|---|---|---|---|---|---|---|---|---|---|---|---|
| Name of Sample | | Triron | PS80 | PS20 | F-68 | DDM | Lec | Tre | Suc | Man | Sor | G-CD | HB-CD | T only |
| Tetanus Toxoid (T) | mg | 9.7 | 9.7 | 9.7 | 9.7 | 9.7 | 9.7 | 9.7 | 9.7 | 9.7 | 9.7 | 9.7 | 9.7 | 9.7 |
| Sodium Chondroitin Sulfate (CS) | mg | 97 | 97 | 97 | 97 | 97 | 97 | 97 | 97 | 97 | 97 | 97 | 97 | |
| Triton X100 | mg | 0.97 | | | | | | | | | | | | |
| Polysorbate 80 | mg | | 0.97 | | | | | | | | | | | |
| Polysorbate 20 | mg | | | 0.97 | | | | | | | | | | |
| Poloxamer 188 | mg | | | | 0.97 | | | | | | | | | |
| N-Dodecyl-B-D-maltoside | mg | | | | | 0.97 | | | | | | | | |
| Lecithin | mg | | | | | | 9.7 | | | | | | | |
| Trehalose | mg | | | | | | | 48.5 | | | | | | |
| Sucrose | mg | | | | | | | | 48.5 | | | | | |
| Mannitol | mg | | | | | | | | | 48.5 | | | | |
| Sorbitol | mg | | | | | | | | | | 48.5 | | | |
| G2-*β*-Cyclodextrin | mg | | | | | | | | | | | 48.5 | | |
| HP-*β*-Cyclodextrin | mg | | | | | | | | | | | | 48.5 | |

### (Example 7)

### Sample Preparation:

A solution was prepared by mixing and dissolving 100 µl of a norovirus VLP (G1) solution (4.4 mg/ml) and 5 mg of sodium chondroitin sulfate (CS). To the thus obtained norovirus VLP-CS mixed solution, 10 mg of trehalose was added and well mixed, and the resultant was dividedly poured into three plastic tubes in an amount of 25 µl each. One of the tubes was used as an initial solution sample, and another was air dried to be used as an initial solid sample.

The other was air dried and then sealed in an aluminum pouch to be stored under conditions of 40°C and 75%RH for 1 week and was used as a solid sample stored at 40°C for 1 week.

### Evaluation:

Water was added in an amount of 175 µl to the initial solution sample, and in an amount of 200 µl to each of the initial solid sample and the solid sample stored at 40°C for 1 week, and the resultants were well mixed.

Evaluation was performed by the size exclusion chromatography. On the basis of a peak area, a VLP content was evaluated.

Results: Results are shown in Table 3.

### [Table 3]

**Table 3**

| | | |
|---|---|---|
| VLP Content (%) | Initial Liquid | 89.8 |
| | Initial Solid | 83.8 |
| | 40°C/1W Solid | 75.2 |

### (Example 8)

### Sample Preparation:

A solution was prepared by mixing and dissolving 100 µl of a norovirus VLP (G1) solution (4.4 mg/ml) and 5 mg of sodium chondroitin sulfate (CS). To the thus obtained norovirus VLP-CS mixed solution, 10 mg of sucrose was added and well mixed, and the resultant was dividedly poured into three plastic tubes in an amount of 25 µl each. One of the tubes was used as an initial solution sample, and another was air dried to be used as an initial solid sample. The other was air dried and then sealed in an aluminum pouch to be stored under conditions of 40°C and 75%RH for 1 week and was used as a solid sample stored at 40°C for 1 week.

Evaluation: The evaluation was performed by the size exclusion chromatography in the same manner as in Example 7.

Results: Results are shown in Table 4.

### [Table 4]

**Table 4**

| | | |
|---|---|---|
| VLP Content (%) | Initial Liquid | 92.1 |
| | Initial Solid | 88.7 |
| | 40°C/1W Solid | 71.1 |

### (Example 9)

### Sample Preparation:

A solution was prepared by mixing and dissolving 100 µl of a norovirus VLP (G1) solution (4.4 mg/ml) and 5 mg of sodium chondroitin sulfate (CS). To the thus obtained norovirus VLP-CS mixed solution, 10 mg of sorbitol was added and well mixed, and the resultant was dividedly poured into three plastic tubes in an amount of 25 µl each. One of the tubes was used as an initial solution sample, and another was air dried to be used as an initial solid sample. The other was air dried and then sealed in an aluminum pouch to be stored under conditions of 40°C and 75%RH for 1 week and was used as a solid sample stored at 40°C for 1 week.

Evaluation: The evaluation was performed by the size exclusion chromatography in the same manner as in Example 7.

Results: Results are shown in Table 5.

### [Table 5]

**Table 5**

| | | |
|---|---|---|
| VLP Content (%) | Initial Liquid | 88.6 |
| | Initial Solid | 85.5 |
| | 40°C/1W Solid | 66.3 |

### (Comparative Example 3)

### Sample Preparation:

A solution was prepared by mixing and dissolving 100 µl of a norovirus VLP (G1) solution (4.4 mg/ml) and 5 mg of sodium chondroitin sulfate (CS). The thus obtained norovirus VLP-CS mixed solution was dividedly poured into three plastic tubes in an amount of 25 µl each. One of the tubes was used as an initial solution sample, and another was air dried to be used as an initial solid sample. The other was air dried and then sealed in an aluminum pouch to be stored under conditions of 40°C and 75%RH for 1 week and was used as a solid sample stored at 40°C for 1 week.

Evaluation: The evaluation was performed in the same manner as in Example 7.

Results: Results are shown in Table 6.

### [Table 6]

**Table 6**

| | | |
|---|---|---|
| VLP Content (%) | Initial Liquid | 73.7 |
| | Initial Solid | 15.4 |
| | 40°C/1 W Solid | 1.0 |

### (Comparative Example 4)

### Sample Preparation:

A norovirus VLP (G1) solution (4.4 mg/ml) was dividedly poured into three plastic tubes in an amount of 25 µl each. One of the tubes was used as an initial solution sample, and another was air dried to be used as an initial solid sample. The other was air dried and then sealed in an aluminum pouch to be stored under conditions of 40°C and 75%RH for 1 week and was used as a solid sample stored at 40°C for 1 week.

Evaluation: The evaluation was performed in the same manner as in Example 7.

Results: Results are shown in Table 7.

### [Table 7]

**Table 7**

| | | |
|---|---|---|
| VLP Content (%) | Initial Liquid | 105.1 |
| | Initial Solid | 4.7 |
| | 40°C/1W Solid | 0.8 |

The norovirus VLP (G1) was more stable in the samples of Examples 7 to 9 than in the samples of Comparative Examples 3 and 4.

### [Table 8]

**Table 8**

| | | Example 7 | Example 8 | Example 9 | Comparative Example 3 | Comparative Example 4 |
|---|---|---|---|---|---|---|
| Norovirus VLP (mg) | | 0.11 | 0.11 | 0.11 | 0.11 | 0.11 |
| Sodium Chondroitin Sulfate (CS) (mg) | | 1.25 | 1.25 | 1.25 | 1.25 | |
| Trehalose (mg) | | 2.5 | | | | |
| Sucrose (mg) | | | 2.5 | | | |
| Sorbitol (mg) | | | | 2.5 | | |
| VLP Content (%) | Initial Liquid | 89.8 | 92.1 | 88.6 | 73.7 | 105.1 |
| | Initial Solid | 83.8 | 88.7 | 85.5 | 15.4 | 4.7 |
| | 40°C/1W Solid | 75.2 | 71.1 | 66.3 | 1.0 | 0.8 |

### (Example 10)

### Sample Preparation:

A solution was prepared by mixing and dissolving 541.8 µl of a norovirus VLP (G1) solution (4.4 mg/ml), 15.5 mg of chitosan glutamate (PROTASAN (Registered Trademark) UP G213) and 158.2 µl of water. To the thus obtained norovirus VLP-chitosan glutamate mixed solution, 58 mg of sucrose was added and well mixed, and the resultant was dividedly poured into plastic tubes in an amount of 10 µl each. One of the tubes was used as an initial solution sample, and the other was air dried and then sealed in an aluminum pouch to be stored under conditions of 40°C and 75%RH for 1 week and was used as a solid sample stored at 40°C for 1 week.

Evaluation: The evaluation was performed by the size exclusion chromatography in the same manner as in Example 7.

Results: Results are shown in Table 9.

### [Table 9]

**Table 9**

| | | |
|---|---|---|
| VLP Content (%) | Initial Liquid | 100 |
| | 40°C/1W Solid | 91 |

### (Comparative Example 5)

### Sample Preparation:

A solution was prepared by mixing and dissolving 541.8 µl of a norovirus VLP (G1) solution (4.4 mg/ml), 15.5 mg of chitosan glutamate (PROTASAN (Registered Trademark) UP G213) and 158.2 µl of water. The thus obtained norovirus VLP-chitosan glutamate mixed solution was dividedly poured into plastic tubes in an amount of 10 µl each. One of the tubes was used as an initial solution sample, and the other was air dried and then sealed in an aluminum pouch to be stored under conditions of 40°C and 75%RH for 1 week and was used as a solid sample stored at 40°C for 1 week.

Evaluation: The evaluation was performed by the size exclusion chromatography in the same manner as in Example 7.

Results: Results are shown in Table 10.

### [Table 10]

**Table 10**

| | | |
|---|---|---|
| VLP Content (%) | Initial Liquid | 100 |
| | 40°C/1W Solid | 34 |

The norovirus VLP (G1) was more stable in the sample of Example 10 than in the sample of Comparative Example 5.

### [Table 11]

**Table 11**

| | | Example 10 | Comparative Example 5 |
|---|---|---|---|
| Norovirus VLP (weight ratio) | | 3 | 12 |
| Chitosan Glutamate (weight ratio) | | 20 | 79 |
| Sucrose (weight ratio) | | 75 | |
| Histidine in Buffer (weight ratio) | | 2 | 9 |
| VLP Content (%) | Initial Liquid | 100 | 100 |
| | 40°C/1W Solid | 91 | 34 |

### (Example 11)

### Sample Preparation:

After 15 mg of a tetanus toxoid antigen, 134.9 mg of sodium chondroitin sulfate (CS) and 0.1 mg of Evans Blue were mixed with and dissolved in water to attain a total amount of 1 ml, bubbles present in the resultant solution were removed under vacuum to obtain a drug filling solution.

A microneedle mold (needle material: SUS316L, needle shape: square pyramid, side length: 300 µm, height: 500 µm, arrangement: 1 mm pitch x 10 columns x 10 rows = 100 needles, square shape, manufactured by Tokai Azumi Techno Co., Ltd.) was heated at 179°C on a heating plate of a mold making tool. A sheet of a styrene-based thermoplastic elastomer (RABARON (Registered Trademark), with a thickness of 1 mm, manufactured by Mitsubishi Chemical Corporation) was cut into a size of about 2.5 cm x 2.5 cm, and the cut sheet was placed over the heated mold and pressed for 30 seconds at a press pressure of about 25 N. The resultant sheet and mold were cooled at room temperature for about 1 minute, and then, the sheet was peeled off from the mold to obtain a microneedle shaping mold having recesses each in a square pyramid shape.

The shaping mold was set on an XY stage of a microneedle manufacturing apparatus, and a dispenser (nozzle size: 0.075 mm in diameter) attached to the manufacturing apparatus was used to discharge the drug filling solution into the recesses of the shaping mold (the arrangement of recesses: 1 mm pitch x 10 columns x 10 rows = 100 recesses). After discharging, the air press was performed for 60 seconds by using a pneumatic press for filling the drug filling solution into the innermost portion of each recess. Thereafter, the shaping mold was dried at room temperature for about 18 hours, and then an acrylic surface of a double sided adhesive tape (No. 5302A, manufactured by Nitto Denko Corporation) was applied onto a surface of the shaping mold and was peeled, so as to collect microneedles on a tape adhesive surface. The thus collected microneedles were caused to adhere onto a surface of a polyethylene sheet via the double sided adhesive tape, and thus, a microneedle patch holding the 100 microneedles thereon was obtained. The obtained microneedles were sealed in an aluminum pouch to be stored at 25°C and 60%RH or 40°C and 75%RH for 1 month.

Evaluation: The content of the tetanus toxoid in each of initial and stored microneedle was evaluated by the size exclusion chromatography.

Results: Results are shown in Table 12.

### [Table 12]

**Table 12**

| | | |
|---|---|---|
| Content of Tetanus Toxoid (%) | Initial | 100 |
| | 25°C/1 M Sealed | 68 |
| | 40°C/1M Sealed | 25 |

Evaluation: The appearance of microneedles was pictured with a microscope.

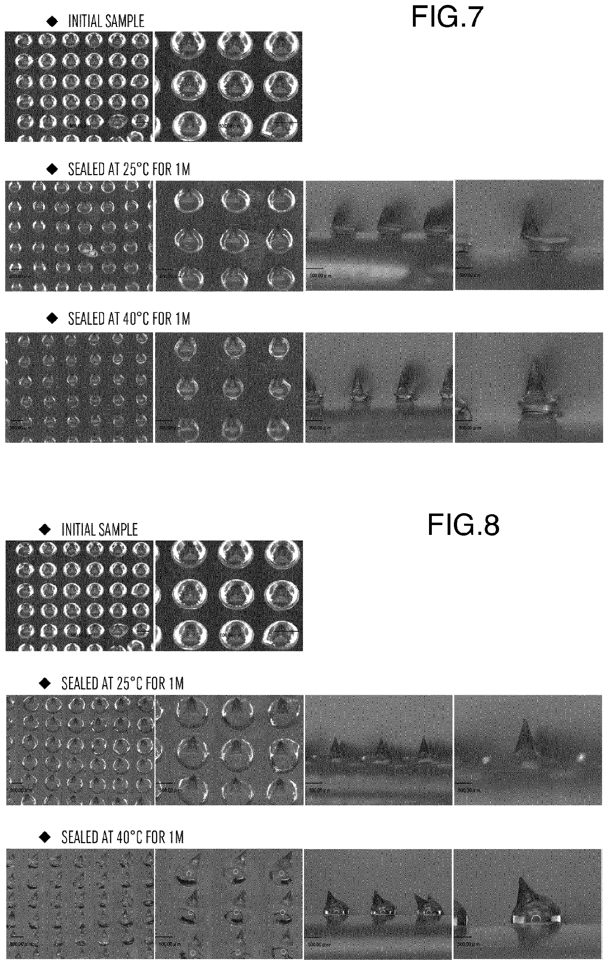

Results: Results are illustrated in Fig. 7.

### (Comparative Example 6)

### Sample Preparation:

After 15 mg of a tetanus toxoid antigen, 134.9 mg of polyvinyl pyrrolidone K-30 (PVP K-30) and 0.1 mg of Evans Blue were mixed with and dissolved in water to attain a total amount of 0.85 ml, bubbles present in the resultant solution were removed under vacuum to obtain a drug filling solution.

A microneedle mold (needle material: SUS316L, needle shape: square pyramid, side length: 300 µm, height: 500 µm, arrangement: 1 mm pitch x 10 columns x 10 rows = 100 needles, square shape, manufactured by Tokai Azumi Techno Co., Ltd.) was heated at 179°C on a heating plate of a mold making tool. A sheet of a styrene-based thermoplastic elastomer (RABARON (Registered Trademark), with a thickness of 1 mm, manufactured by Mitsubishi Chemical Corporation) was cut into a size of about 2.5 cm x 2.5 cm, and the cut sheet was placed over the heated mold and pressed for 30 seconds at a press pressure of about 25 N. The resultant sheet and mold were cooled at room temperature for about 1 minute, and then, the sheet was peeled off from the mold to obtain a microneedle shaping mold having recesses each in a square pyramid shape.

The shaping mold was set on an XY stage of a microneedle manufacturing apparatus, and a dispenser (nozzle size: 0.075 mm in diameter) attached to the manufacturing apparatus was used to discharge the drug filling solution into the recesses of the shaping mold (the arrangement of recesses: 1 mm pitch x 10 columns x 10 rows = 100 recesses). After discharging, the air press was performed for 60 seconds by using a pneumatic press for filling the drug filling solution into the innermost portion of each recess. Thereafter, the shaping mold was dried at room temperature for about 18 hours, and an acrylic surface of a double sided adhesive tape (No. 5302A, manufactured by Nitto Denko Corporation) was applied onto a surface of the shaping mold and was peeled, so as to collect microneedles on a tape adhesive surface. The thus collected microneedles were caused to adhere onto a surface of a polyethylene sheet via the double sided adhesive tape, and thus, a microneedle patch holding the 100 microneedles thereon was obtained. The obtained microneedles were sealed in an aluminum pouch to be stored at 25°C and 60%RH or 40°C and 75%RH for 1 month.

Evaluation: The content of the tetanus toxoid in each of initial and stored microneedle was evaluated by the size exclusion chromatography.

Results: Results are shown in Table 13.

### [Table 13]

**Table 13**

| | | |
|---|---|---|
| Content of Tetanus Toxoid (%) | Initial | 100 |
| | 25°C/1 M Sealed | 55 |
| | 40°C/1 M Sealed | 0 |

Evaluation: The appearance of microneedles was pictured with a microscope.

Results: Results are illustrated in Fig. 8.

The tetanus toxoid was more stable in the sample of Example 11 than in the sample of Comparative Example 6. Besides, based on the micrographs of Figs. 7 and 8, the physical stability in the shapes of the microneedles after the storage was superior in the sample of Example 11 to that in the sample of Comparative Example 6.

### [Table 14]

**Table 14**

| | | Example 11 | Comparative Example 6 |
|---|---|---|---|
| Tetanus Toxoid (weight ratio) | | 10 | 10 |
| Sodium Chondroitin Sulfate (weight ratio) | | 89.9 | |
| Polyvinyl Pyrrolidone K-30 (weight ratio) | | | 89.9 |
| Evans Blue (weight ratio) | | 0.1 | 0.1 |
| Content of Tetanus Toxoid (%) | Initial | 100 | 100 |
| | 25°C/1M Sealed | 68 | 55 |
| | 40°C/1 M Sealed | 25 | 0 |

### (Example 12)

### Sample Preparation:

After mixing 564 µl of a norovirus VLP (G1) solution (VLP: 2.5 mg, NaCl: 0.033 mg, histidine: 1.8 mg) and 484 µl of a norovirus VLP (G2) solution (VLP: 2.5 mg, sucrose: 97 mg, NaCl: 5.7 mg, histidine: 1.5 mg), 25 mg of chitosan glutamate (PROTASAN (Registered Trademark) UP G213) was added to the resulting mixture to be dissolved therein by stirring, and then bubbles present in the resultant solution were removed under vacuum to obtain a filling solution. The weight ratios among principal components in a solid content were norovirus VLP (G1): 1.8%, norovirus VLP (G2): 1.8%, sucrose: 71.3% and chitosan glutamate: 18.4%, and a solid content concentration of the filling solution was set to 11.5% (in weight ratio).

A microneedle mold (needle material: SUS316L, needle shape: square pyramid, side length: 300 µm, height: 500 µm, arrangement: 1 mm pitch x 10 columns x 10 rows = 100 needles, square shape, manufactured by Tokai Azumi Techno Co., Ltd.) was heated at 179°C on a heating plate of a mold making tool (manufactured by Kyokko Seiko Co., Ltd.). A sheet of a styrene-based thermoplastic elastomer (RABARON (Registered Trademark), with a thickness of 1 mm, manufactured by Mitsubishi Chemical Corporation) was cut into a size of about 2.5 cm x 2.5 cm, and the cut sheet was placed over the heated mold and pressed for 30 seconds at a press pressure of about 25 N. The resultant sheet and mold were cooled at room temperature for about 1 minute, and then, the sheet was peeled off from the mold to obtain a microneedle shaping mold having recesses each in a square pyramid shape.

The shaping mold was set on an XY stage of a microneedle manufacturing apparatus (manufactured by Kyokko Seiko Co., Ltd.), and a dispenser 1 (nozzle size: 0.075 mm in diameter) was used to fill the antigen-containing solution in the 100 needle holes up to needle bases (feed pressure: 0.017 MPa, number of times of application: 40 times). After filling, the air press was performed for 60 seconds by using a pneumatic press for filling the polymer into the innermost portion of each hole to remove bubbles remaining in the tip portion of the mold hole. Thereafter, the shaping mold was dried at room temperature for about 18 hours, and then an acrylic surface of a double sided adhesive tape (No. 5302A, manufactured by Nitto Denko Corporation) adhering to a surface of a soft polyethylene support sheet (diameter: 18 mm, thickness: 0.3 mm) was applied onto a surface of the polymer and was peeled, so as to collect microneedles on a tape adhesive surface. The thus obtained microneedles were put in an aluminum bag of 200 x 150 mm together with one piece of synthetic zeolite desiccant (synthetic zeolite 4A, 10 g, 60 x 40 x 4 mm), and the resultant bag was heat sealed. Aluminum bags each containing the preparation sealed therein were stored in a refrigerator at 5°C or a thermo-hygrostat at 25°C/60%RH or 40°C/75%RH. After 3 months, the microneedle patches were collected from the aluminum bags.

Evaluation: The appearance of the stored microneedles was observed with a digital microscope.

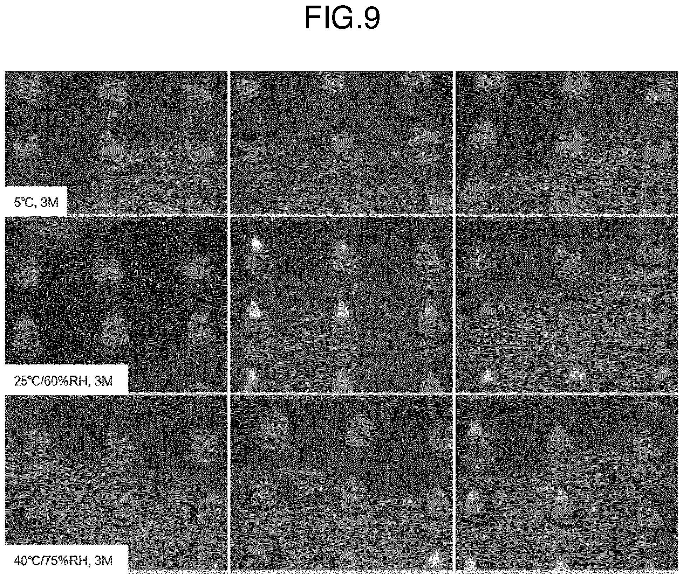

Results: Results are illustrated in Fig. 9.

Evaluation: The content of VLP in each preparation was measured by the size exclusion chromatography.

Results: Results are shown in Table 15.

### [Table 15]

**Table 15**

| | | |
|---|---|---|
| VLP Content (%) | Initial | 100 |
| | 5°C (sealed with desiccant)/3M Stored | 91 |
| | 25°C/60%RH (sealed with desiccant)/3M Stored | 94 |
| | 40°C/75%RH (sealed with desiccant)/3M Stored | 96 |

Evaluation: The contents of G1 and G2 proteins in each preparation were measured by the reverse phase chromatography.

Results: Results are shown in Table 16.

### [Table 16]

**Table 16**

| | | |
|---|---|---|
| Content of G1 Protein (%) | Initial | 100 |
| | 5°C (sealed with desiccant)/3M Stored | 91 |
| | 25°C/60%RH (sealed with desiccant)/3M Stored | 99 |
| | 40°C/75%RH (sealed with desiccant)/3M Stored | 98 |
| Content of G2 Protein (%) | Initial | 100 |
| | 5°C (sealed with desiccant)/3M Stored | 93 |
| | 25°C/60%RH (sealed with desiccant)/3M Stored | 99 |
| | 40°C/75%RH (sealed with desiccant)/3M Stored | 100 |

### (Example 13)

### Sample Preparation:

After mixing 1693 µl of a norovirus VLP (G1) solution (VLP: 7.5 mg, NaCl: 0.099 mg, histidine: 5.3 mg) and 1453 µl of a norovirus VLP (G2) solution (VLP: 7.5 mg, sucrose: 291 mg, NaCl: 17 mg, histidine: 4.5 mg), 75 mg of chitosan glutamate (PROTASAN (Registered Trademark) UP G213) was added to the resulting mixture to be dissolved therein by stirring, and then bubbles present in the resultant solution were removed under vacuum to obtain an antigen filling solution. The weight ratios among principal components in a solid content were norovirus VLP (G1): 1.8%, norovirus VLP (G2): 1.8%, sucrose: 71.3% and chitosan glutamate: 18.4%, and a solid content concentration of the filling solution was set to 11.5% (in weight ratio). Besides, 80 mg of chitosan glutamate and 320 mg of sucrose were dissolved in 3200 µl of distilled water to obtain a base filling solution.

A microneedle mold (needle material: SUS316L, needle shape: square pyramid, side length: 300 µm, height: 500 µm, arrangement: 1 mm pitch x 10 columns x 10 rows = 100 needles, square shape, manufactured by Tokai Azumi Techno Co., Ltd.) was heated at 179°C on a heating plate of a mold making tool (manufactured by Kyokko Seiko Co., Ltd.). A sheet of a styrene-based thermoplastic elastomer (RABARON (R), with a thickness of 1 mm, manufactured by Mitsubishi Chemical Corporation) was cut into a size of about 2.5 cm x 2.5 cm, and the cut sheet was placed over the heated mold and pressed for 30 seconds at a press pressure of about 25 N. After about 1 minute, the sheet was peeled off from the mold to obtain a microneedle shaping mold.

The shaping mold was set on an XY stage of a microneedle manufacturing apparatus (manufactured by Kyokko Seiko Co., Ltd.), and a dispenser 1 (nozzle size: 0.075 mm in diameter) was used to fill the antigen-containing solution in the 100 needle holes continuously by 24 times (feed pressure: 0.017 MPa). After filling, the air press was performed for 60 seconds by using a pneumatic press for filling the polymer into the innermost portion of each hole to remove bubbles remaining in the tip portion of the mold hole. Subsequently, the base filling solution was filled in base portions by 18 times, and the air press was similarly performed. Thereafter, the shaping mold was dried at room temperature for about 18 hours, and then an acrylic surface of a double sided adhesive tape (No. 5302A, manufactured by Nitto Denko Corporation) adhering to a surface of a soft polyethylene support sheet (diameter: 18 mm, thickness: 0.3 mm) was applied onto a surface of the polymer and was peeled, so as to collect microneedles on a tape adhesive surface. The thus obtained microneedles were put in an aluminum bag of 200 x 150 mm together with one piece of synthetic zeolite desiccant (synthetic zeolite 4A, 10 g, 60 x 40 x 4 mm), and the resultant bag was heat sealed. Aluminum bags each containing the preparation sealed therein were stored in a refrigerator at 5°C or a thermo-hygrostat at 25°C/60%RH or 40°C/75%RH. After 1 month, the microneedle patches were collected from the aluminum bags.

Evaluation: The appearance of the stored microneedles was observed with a digital microscope.

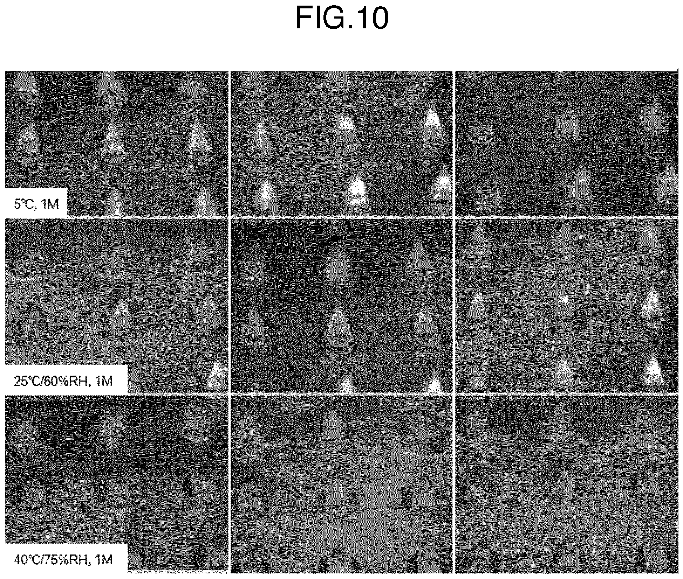

Results: Results are illustrated in Fig. 10.

Evaluation: The content of VLP in each preparation was measured by the size exclusion chromatography.

Results: Results are shown in Table 17.

### [Table 17]

**Table 17**

| | | |
|---|---|---|
| VLP Content (%) | Initial | 100 |
| | 5°C (sealed with desiccant)/1 M Stored | 95 |
| | 25°C/60%RH (sealed with desiccant)/1 M Stored | 96 |
| | 40°C/75%RH (sealed with desiccant)/1 M Stored | 95 |

Evaluation: A rabbit (kbl/NZWN, female, 8 weeks old) was administered with one or two microneedle patches (containing G1 and G2 proteins each in 20 µg/patch). Twenty-one days after the first administration, the second additional administration was performed at the same dose as in the first administration. Twenty-one days after the second administration, blood was collected to measure G1- and G2-specific IgG and IgA in the blood serum by ELISA.

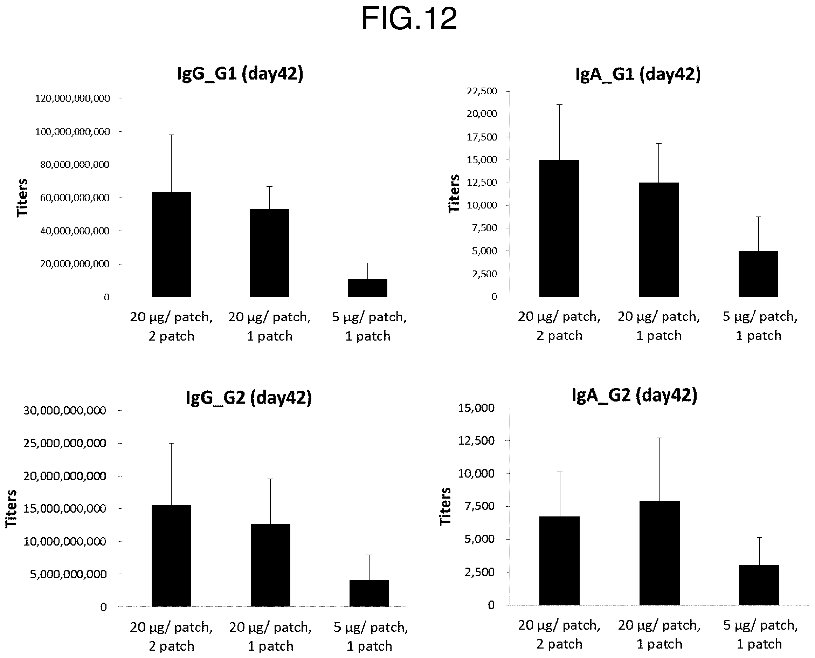

Results: Results are illustrated in Fig. 12.

### (Example 14)

### Sample Preparation:

After mixing 1693 µl of a norovirus VLP (G1) solution (VLP: 7.5 mg, NaCl: 0.099 mg, histidine: 5.3 mg) and 1453 µl of a norovirus VLP (G2) solution (VLP: 7.5 mg, sucrose: 291 mg, NaCl: 17 mg, histidine: 4.5 mg), 75 mg of chitosan glutamate (PROTASAN (Registered Trademark) UP G213) was added to the resulting mixture to be dissolved therein by stirring, and then bubbles present in the resultant solution were removed under vacuum to obtain an antigen filling solution. The weight ratios among principal components in a solid content were norovirus VLP (G1): 1.8%, norovirus VLP (G2): 1.8%, sucrose: 71.3% and chitosan glutamate: 18.4%, and a solid content concentration of the filling solution was set to 11.5% (in weight ratio). Besides, 80 mg of chitosan glutamate and 320 mg of sucrose were dissolved in 3200 µl of distilled water to obtain a base filling solution.

A microneedle mold (needle material: SUS316L, needle shape: square pyramid, side length: 300 µm, height: 500 µm, arrangement: 1 mm pitch x 10 columns x 10 rows = 100 needles, square shape, manufactured by Tokai Azumi Techno Co., Ltd.) was heated at 179°C on a heating plate of a mold making tool (manufactured by Kyokko Seiki Co., Ltd.). A sheet of a styrene-based thermoplastic elastomer (RABARON (Registered Trademark), with a thickness of 1 mm, manufactured by Mitsubishi Chemical Corporation) was cut into a size of about 2.5 cm x 2.5 cm, and the cut sheet was placed over the heated mold and pressed for 30 seconds at a press pressure of about 25 N. After about 1 minute, the sheet was peeled off from the mold to obtain a microneedle shaping mold.

The shaping mold was set on an XY stage of a microneedle manufacturing apparatus (manufactured by Kyokko Seiko Co., Ltd.), and a dispenser 1 (nozzle size: 0.075 mm in diameter) was used to fill the antigen-containing solution in the 100 needle holes continuously by 6 times (feed pressure: 0.017 MPa). After filling, the air press was performed for 60 seconds by using a pneumatic press for filling the polymer into the innermost portion of each hole to remove bubbles remaining in the tip portion of the mold hole. Subsequently, the base filling solution was filled in base portions by 36 times, and the air press was similarly performed. Thereafter, the shaping mold was dried at room temperature for about 18 hours, and then an acrylic surface of a double sided adhesive tape (No. 5302A, manufactured by Nitto Denko Corporation) adhering to a surface of a soft polyethylene support sheet (diameter: 18 mm, thickness: 0.3 mm) was applied onto a surface of the polymer and was peeled, so as to collect microneedles on a tape adhesive surface. The thus obtained microneedles were put in an aluminum bag of 200 x 150 mm together with one piece of synthetic zeolite desiccant (synthetic zeolite 4A, 10 g, 60 x 40 x 4 mm), and the resultant bag was heat sealed. Aluminum bags each containing the preparation sealed therein were stored in a refrigerator at 5°C or a thermo-hygrostat at 25°C/60%RH or 40°C/75%RH. After 1 month, the microneedle patches were collected from the aluminum bags.

Evaluation: The appearance of the stored microneedles was observed with a digital microscope.

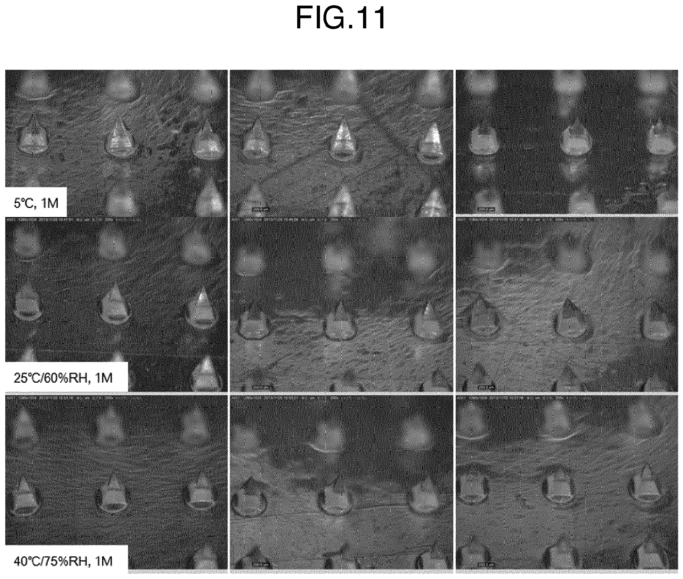

Results: Results are illustrated in Fig. 11.

Evaluation: The content of VLP in each preparation was measured by the size exclusion chromatography.

Results: Results are shown in Table 18.

### [Table 18]

**Table 18**

| | | |
|---|---|---|
| VLP Content (%) | Initial | 100 |
| | 5°C (sealed with desiccant)/1M Stored | 92 |
| | 25°C/60%RH (sealed with desiccant)/1 M Stored | 108 |
| | 40°C/75%RH (sealed with desiccant)/1 M Stored | 123 |

Evaluation: A rabbit (kbl/NZWN, female, 8 weeks old) was administered with one microneedle patch (containing G1 and G2 proteins each in 5 µg/patch). Twenty-one days after the first administration, the second additional administration was performed at the same dose as in the first administration. Twenty-one days after the second administration, blood was collected to measure G1- and G2-specific IgG and IgA in the blood serum by the ELISA.

Results: Results are illustrated in Fig. 12.

In Example 12, it was revealed that the norovirus proteins (G1 and G2) had stability. In the samples of Examples 12 to 14, it was revealed that the norovirus VLPs (G1 and G2) had also stability. Besides, on the basis of the micrographs of Figs. 9 to 11, the physical stability in the shapes of the stored microneedles was observed also in the samples of Examples 12 to 14.

In Examples 13 and 14, antibody titers were measured in accordance with the amount per patch of the antigen of the norovirus proteins (G1 and G2) in the microneedles and the number of administered patches. It was revealed based on these results that the microneedles of Examples 13 and 14 have an ability to induce an immune response when intradermally administered.

### Industrial Applicability

A microneedle of the present invention is used for more efficiently preventing or treating infectious diseases such as tetanus, diphtheria, norovirus, Dengue fever, human papilloma virus (HPV), influenza and rotavirus, and other diseases. Accordingly, the present invention makes a great contribution to the development of medical device industry and related industries.

### Reference Signs List

1 preparation
2 microspike
3 base (support)
4 projection base
5 base (spike holding member)
6 support

## Claims

1. A preparation having a soluble microspike, the microspike containing a vaccine antigen, an ionic polymer base material, and at least one agent selected from the group consisting of a non-reducing sugar, a sugar alcohol, a cyclodextrin and a surfactant, wherein the ionic polymer base material contains a polysaccharide, and the polysaccharide is at least one selected from the group consisting of chondroitin sulfuric acid, hyaluronic acid, chitosan and chitin, and salts thereof, and wherein the surfactant is at least one selected from the group consisting of a nonionic surfactant and a lecithin.

2. The preparation according to claim 1, wherein an amount of the at least one selected from the group consisting of a non-reducing sugar, a sugar alcohol, a cyclodextrin and a surfactant is 0.01 to 94.99% by weight of the entire soluble microspike.

3. The preparation according to claim 1, wherein an amount of the vaccine antigen is 0.01 to 10% by weight of the entire soluble microspike.

4. The preparation according to claim 1, wherein an amount of the ionic polymer base material is 1 to 99.98% by weight of the entire soluble microspike.

5. The preparation according to claim 1, wherein degradation or aggregation of the vaccine antigen occurring during drying or storage of the preparation is suppressed to improve biological stability.

6. The preparation according to claim 1, wherein the soluble microspike is soluble in vivo.

7. The preparation according to claim 1, wherein the vaccine antigen is at least one toxoid selected from the group consisting of a tetanus toxoid and a diphtheria toxoid.

8. The preparation according to claim 1, wherein the vaccine antigen is at least one vaccine antigen having a particulate structure selected from the group of norovirus vaccine, a Dengue fever vaccine, an HPV vaccine, an influenza vaccine and a rotavirus vaccine.

9. The preparation according to claim 1, wherein the non-reducing sugar and the sugar alcohol are at least one selected from the group consisting of trehalose, sucrose, mannitol and sorbitol.

10. The preparation according to claim 1, wherein the cyclodextrin is at least one selected from the group consisting of HP-β-cyclodextrin and G2-β-cyclodextrin.

11. The preparation according to any one of claims 1 to 10, wherein a projection including the soluble microspike is directly held on a support, or wherein a projection including the soluble microspike is held on a base to form a spike holding member, and the spike holding member is held on a support.

12. A method for stabilizing a preparation having a soluble microspike, wherein at least one selected from the group consisting of a non-reducing sugar, a sugar alcohol, a cyclodextrin and a surfactant, and an ionic polymer base material are contained in the soluble microspike containing a vaccine antigen.

## Patentansprüche

1. Zubereitung, die einen löslichen Mikrospike aufweist, wobei der Mikrospike ein Vakzin-Antigen, ein ionisches Polymerbasismaterial und mindestens ein Mittel ausgewählt aus der Gruppe bestehend aus einem nichtreduzierenden Zucker, einem Zuckeralkohol, einem Cyclodextrin und einem Tensid enthält, wobei das ionische Polymerbasismaterial ein Polysaccharid enthält, und das Polysaccharid mindestens eines ausgewählt aus der Gruppe bestehend aus Chondroitin-Schwefelsäure, Hyaluronsäure, Chitosan und Chitin und Salzen davon ist und wobei das Tensid mindestens eines ausgewählt aus der Gruppe bestehend aus einem nichtionischen Tensid und einem Lecithin ist.

2. Zubereitung nach Anspruch 1, wobei eine Menge des mindestens einen ausgewählt aus der Gruppe bestehend aus einem nicht reduzierenden Zucker, einem Zuckeralkohol, einem Cyclodextrin und einem Tensid 0,01 bis 94,99 Gew.-% des gesamten löslichen Mikrospikes beträgt.

3. Zubereitung nach Anspruch 1, wobei die Menge des Vakzin-Antigens 0,01 bis 10 Gew.-% des gesamten löslichen Mikrospikes beträgt.

4. Zubereitung nach Anspruch 1, wobei die Menge des ionischen Polymerbasismaterials 1 bis 99,98 Gew.-% des gesamten löslichen Mikrospikes beträgt.

5. Zubereitung nach Anspruch 1, wobei Zerfall oder Aggregation des Vakzin-Antigens, auftretend während des Trocknens oder der Lagerung der Zubereitung, zur Verbesserung der biologischen Stabilität unterdrückt worden ist.

6. Zubereitung nach Anspruch 1, wobei der lösliche Mikrospike *in vivo* löslich ist.

7. Zubereitung nach Anspruch 1, wobei das Vakzin-Antigen mindestens ein Toxoid ausgewählt aus der Gruppe bestehend aus einem Tetanus-Toxoid und einem Diphtherietoxoid ist.

8. Zubereitung nach Anspruch 1, wobei das Vakzin-Antigen mindestens ein Vakzin-Antigen mit einer partikelförmigen Struktur ist, ausgewählt aus der Gruppe von Norovirus-Vakzin, einem Dengue-Fieber-Vakzin, einem HPV-Vakzin, einem Influenza-Vakzin und einem Rotavirus-Vakzin.

9. Zubereitung nach Anspruch 1, wobei der nicht reduzierende Zucker und der Zuckeralkohol mindestens einer ausgewählt aus der Gruppe bestehend aus Trehalose, Saccharose, Mannit und Sorbit ist.

10. Zubereitung nach Anspruch 1, wobei das Cyclodextrin mindestens eines ausgewählt aus der Gruppe bestehend aus HP-β-Cyclodextrin und G2-β-Cyclodextrin ist.

11. Zubereitung nach einem der Ansprüche 1 bis 10, wobei eine Auskragung, die den löslichen Mikrospike enthält, direkt auf einem Träger gehalten wird, oder wobei eine Auskragung, die den löslichen Mikrospike enthält, auf einer Basis gehalten wird, um ein Spike-Halteelement zu bilden und das Spike-Halteelement auf einem Träger gehalten wird.

12. Verfahren zur Stabilisierung einer Zubereitung, die einen löslichen Mikrospike aufweist, wobei mindestens eines ausgewählt aus der Gruppe bestehend aus einem nicht reduzierenden Zucker, einem Zuckeralkohol, einem Cyclodextrin und einem Tensid und einem ionischen Polymerbasismaterial, in dem löslichen Mikrospike, der ein Vakzin-Antigen enthält, enthalten ist.

## Revendications

1. Préparation comportant une micropointe soluble, la micropointe contenant un antigène vaccinant, un matériau de base polymère ionique, et au moins un agent choisi dans l'ensemble constitué par un sucre non réducteur, un alcool glucidique, une cyclodextrine et un tensioactif, dans laquelle le matériau de base polymère ionique contient un polysaccharide, et le polysaccharide est au moins un polysaccharide choisi dans l'ensemble constitué par le hyaluronate, sulfate de chondroïtine, le chitosane et la chitine, et leurs sels, et dans laquelle le tensioactif est au moins un tensioactif choisi dans l'ensemble constitué par un tensioactif non ionique et une lécithine.

2. Préparation selon la revendication 1, dans laquelle une quantité dudit au moins un agent choisi dans l'ensemble constitué par un sucre non réducteur, un alcool glucidique, une cyclodextrine et un tensioactif vaut de 0,01 à 94,99 % en poids de la micropointe soluble entière.

3. Préparation selon la revendication 1, dans laquelle une quantité de l'antigène vaccinant vaut de 0,01 à 10 % en poids de la micropointe soluble entière.

4. Préparation selon la revendication 1, dans laquelle une quantité du matériau de base polymère ionique vaut de 1 à 99,98 % en poids de la micropointe soluble entière.

5. Préparation selon la revendication 1, dans laquelle la dégradation ou l'agrégation de l'antigène vaccinant, ayant lieu au cours du séchage ou du stockage de la préparation, est supprimée pour améliorer la stabilité biologique.

6. Préparation selon la revendication 1, dans laquelle la micropointe soluble est soluble in vivo.

7. Préparation selon la revendication 1, dans laquelle l'antigène vaccinant est au moins un toxoïde choisi dans l'ensemble constitué par un toxoïde tétanique et un toxoïde diphtérique.

8. Préparation selon la revendication 1, dans laquelle l'antigène vaccinant est au moins un antigène vaccinant ayant une structure particulaire choisie dans l'ensemble constitué par un vaccin contre les norovirus, un vaccin contre la dengue, un vaccin contre le PVH, un vaccin contre la grippe et un vaccin contre les rotavirus.

9. Préparation selon la revendication 1, dans laquelle le sucre non réducteur et l'alcool glucidique consistent en au moins un choisi dans l'ensemble constitué par le tréhalose, le saccharose, le mannitol et le sorbitol.

10. Préparation selon la revendication 1, dans laquelle la cyclodextrine est au moins une choisie dans l'ensemble constitué par la HP-β-cyclodextrine et la G2-β-cyclodextrine.

11. Préparation selon l'une quelconque des revendications 1 à 10, dans laquelle une projection incluant la micropointe soluble est directement maintenue sur un support, ou dans laquelle une projection incluant la micropointe soluble est maintenue sur une base pour former un élément portant la pointe, et l'élément portant la pointe est maintenu sur un support.

12. Procédé pour stabiliser une préparation comportant une micropointe soluble, dans lequel au moins un agent choisi dans l'ensemble constitué par un sucre non réducteur, un alcool glucidique, une cyclodextrine et un tensioactif, et un matériau de base polymère ionique sont contenus dans la micropointe soluble contenant un antigène vaccinant.
